# EUROPEAN PATENT SPECIFICATION

(11) **EP 2 062 874 B1**
(45) Date of publication and mention of the grant of the patent: **17.12.2014**
(21) Application number: 07022481.1
(22) Date of filing: 20.11.2007
(51) Int. Cl.: C07C 231/12, C07C 247/04, C07C 57/03, C07C 59/42, C07D 307/32, C07D 313/00, C07F 7/18, A61K 31/165, A61P 9/12

(54) **Process and intermediates for the preparation of aliskiren**
Verfahren und Zwischenprodukte für die Herstellung von Aliskiren
Procédé et intermédiaires pour la préparation d'aliskiren

(43) Date of publication of application: 27.05.2009
(73) Proprietor: KRKA, tovarna zdravil, d.d., Novo mesto, 8501 Novo mesto (SI)
(72) Inventor: Kidemet, Davor, 42000 Varazdin (HR); Zupet, Rok, 1211 Ljubljana (SI)
(74) Representative: UEXKÜLL & STOLBERG

(56) References cited:
- EP-A- 0 678 503
- EP-A- 0 678 514
- WO-A-01/09083
- WO-A-02/02508
- US-A1- 2006 018 960

## Description

The present invention relates to a process and intermediates for the manufacture of a compound having the following formula (I) : 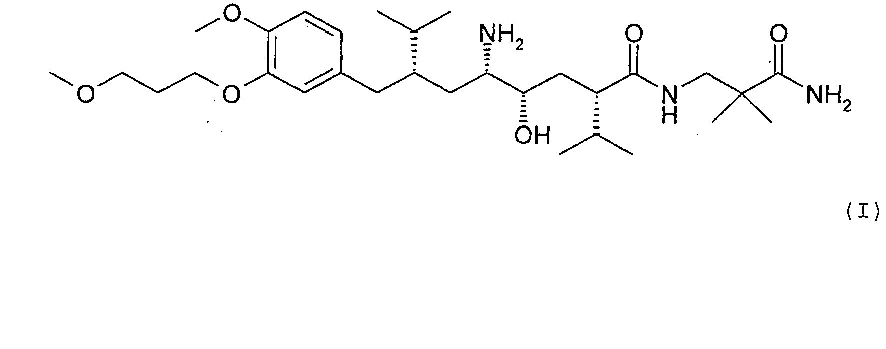 i.e. (2S,4S,5S,7S)-5-amino-N-(2-carbamoyl-2-methylpropyl)-4-hydroxy-2-isopropyl-7-[4-methoxy-3-(3-methoxypropoxy)benzyl]-8-methylnonanamide (hereinafter referred to by its generic name "aliskiren"), or pharmaceutically acceptable salts thereof.

Aliskiren is an orally effective renin inhibitor. It is useful in the treatment of hypertension and its hemifumarate salt is commercially available under the trade names Tekturna^{®} and Rasilez^{®}.

The synthesis of 2(S), 4(S), 5(S), 7(S)-2,7-dialkyl-4-hydoxy-5-amino-8-aryl-octanoyl amides such as aliskiren is known from EP-A-0 678 503. Further multistage syntheses of substituted octanoyl amides are for example described in EP-A-1 215 201, WO-A-01/09079, WO-A-01/09083, WO-A-02/02508, WO-A-02/08172, WO-A-03/103653, WO-A-2006/024501, WO-A-2006/131304, WO-A-2007/039183, GB-A-2 431 640, GB-A-2 431 641, GB-A-2 431 642, GB-A-2 431 643, GB-A-2 431 644, GB-A-2 431 645, GB-A-2 431 646, GB-A-2 431 647, GB-A-2 431 648, GB-A-2 431 649, GB-A-2 431 650, GB-A-2 431 651, GB-A-2 431 652, GB-A-2 431 653 and GB-A-2 431 654.EP-A-0 678 515 describes 3,5-disubstituted tetrahydrofuran-2-ones as synthesis intermediates.

Nevertheless, there is still a need for a process for preparing aliskiren which process is characterized in that aliskiren is obtainable in a high total yield and in a high degree of diastereomeric and enantiomeric purity.

This object is surprisingly solved by a process for the manufacture of aliskiren according to any one of claims 1 to 33 and 44. The invention is also directed to the compounds according to claims 34 to 42 as well as their use according to claim 43 and a process according to claims 44 and 45.

The process according to the invention for preparing aliskiren of the following formula (I) or pharmaceutically acceptable salts thereof 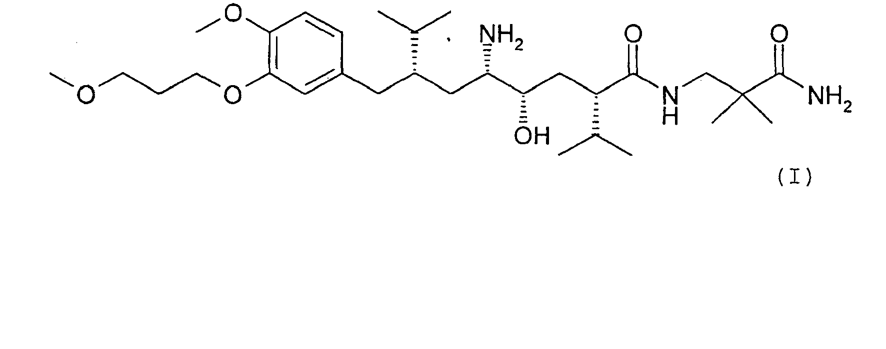 comprises
(a) converting a compound of formula (II) 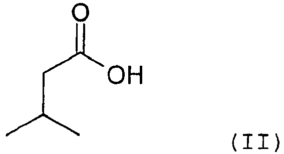 into a compound of formula (III) 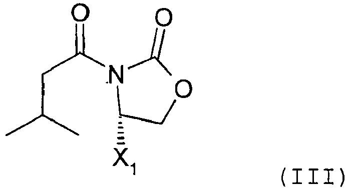 wherein X₁ is an alkylaryl group,
(b) reacting the compound of formula (III) with 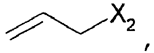 wherein X₂ is halogen,
   to form a compound of formula (IV) 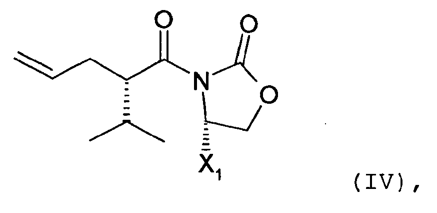
(c) converting the compound of formula (IV) into a compound of formula (V) 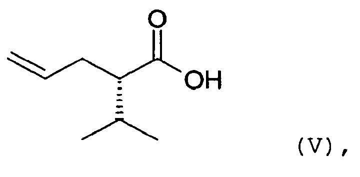
(d) converting the compound of formula (V) into a compound of formula (VI) 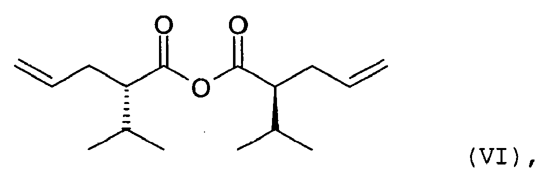
(e) cyclizing the compound of formula (VI) in the presence of a metal catalyst to form a compound of formula (VII) 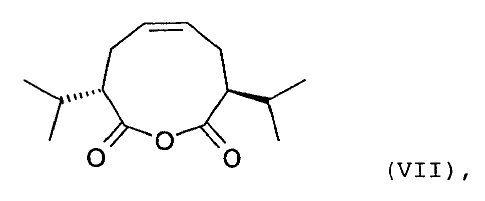
(f) converting the compound of formula (VII) into a compound of formula (VIII) or (VIIIa) 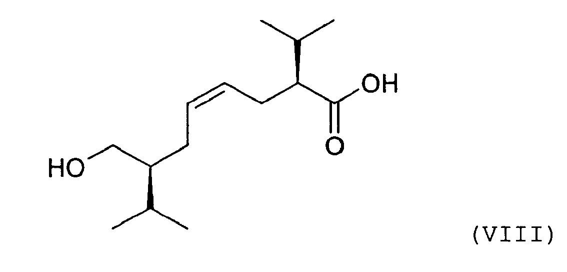 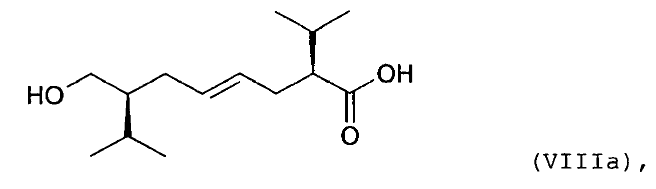
(g) lactonizing the compound of formula (VIII) or (VIIIa) into a compound of formula (IX) 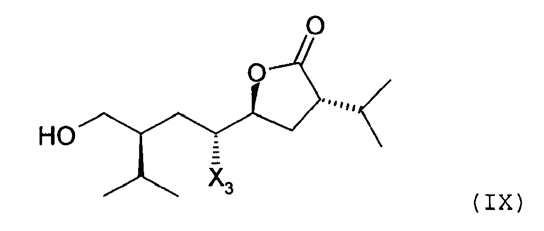 wherein X₃ is halogen,
(h) azidating the compound of formula (IX) to form a compound of formula (X) 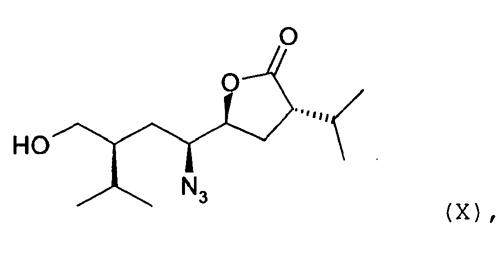 and either
(i1) converting the compound of formula (X) into a compound of formula (XI) 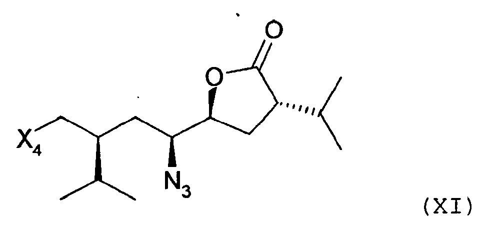 wherein X₄ is halogen,
(j1) reacting the compound of formula (XI) with a compound of formula (XII) in the presence of a metal catalyst 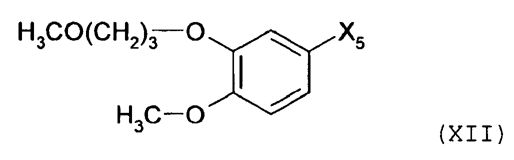 wherein X₅ is halogen,
   to form a compound of formula (XIII) 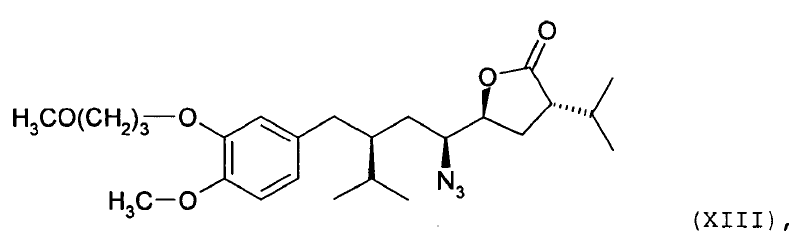
(k1) reacting the compound of formula (XIII) with an amine of formula (XIV) 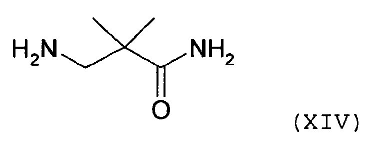 to form a compound of formula (XV) 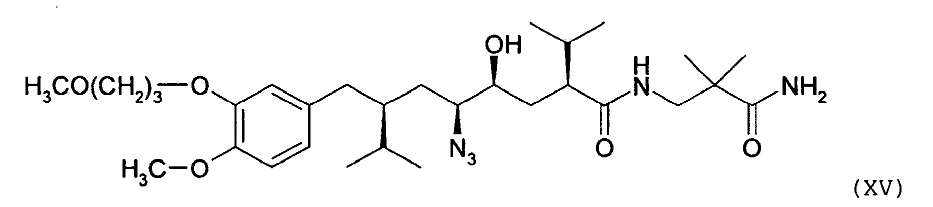 and
(l1) converting the compound of formula (XV) into the compound of formula (I) or a pharmaceutically acceptable salt thereof,
   or
(i2) converting the compound of formula (X) into a compound of formula (XVI) 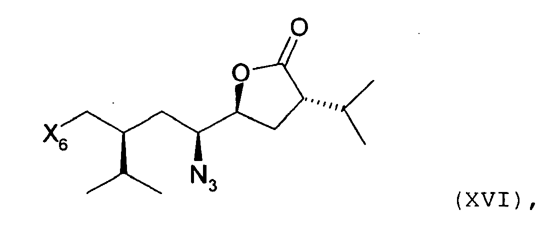 wherein X₆ is selected from the group consisting of trialkylsilyl, dialkylarylsilyl, alkyldiarylsilyl and triarylsilyl,
(j2) reacting the compound of formula (XVI) with an amine of formula (XIV) 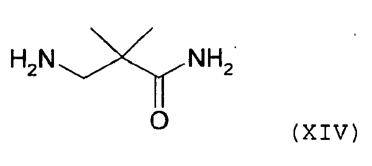 to form a compound of formula (XVII) 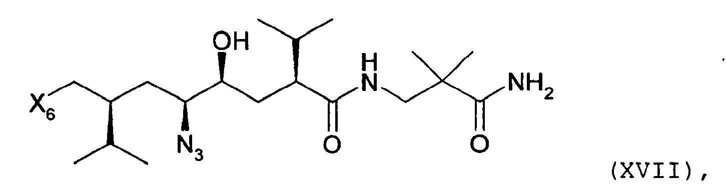
(k2) converting the compound of formula (XVII) into a compound of formula (XVIII) 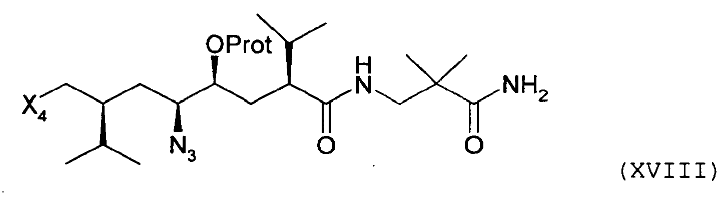 wherein X₄ is halogen and Prot is a hydroxyl protecting group,
(12) reacting the compound of formula (XVIII) with a compound of formula (XII) in the presence of a metal catalyst 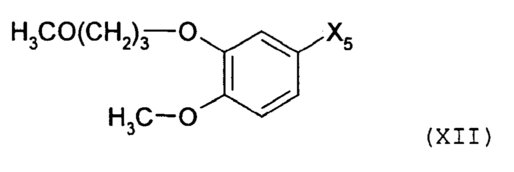 wherein X₅ is halogen,
   to form a compound of formula (XIX) 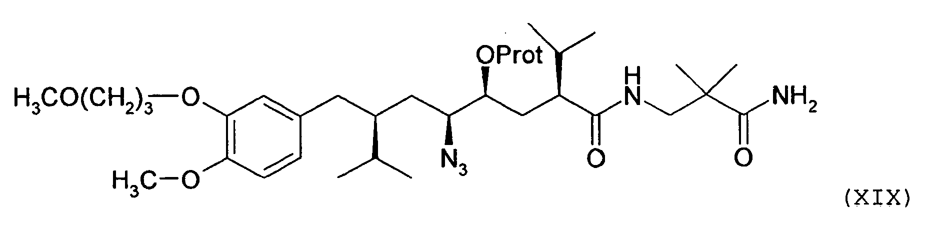 and
(m2) converting the compound of formula (XIX) into the compound of formula (I) or a pharmaceutically acceptable salt thereof.

Thus, according to the first option of the invention the process for the manufacture of aliskiren can be characterized by reactions steps as depicted in schemes 1 and 2 below.

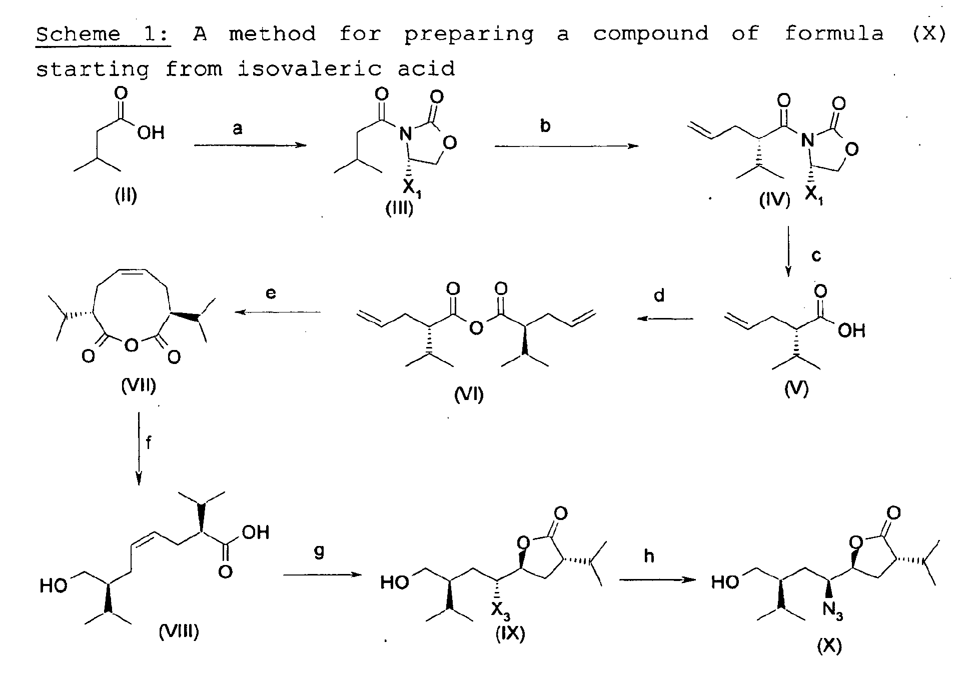

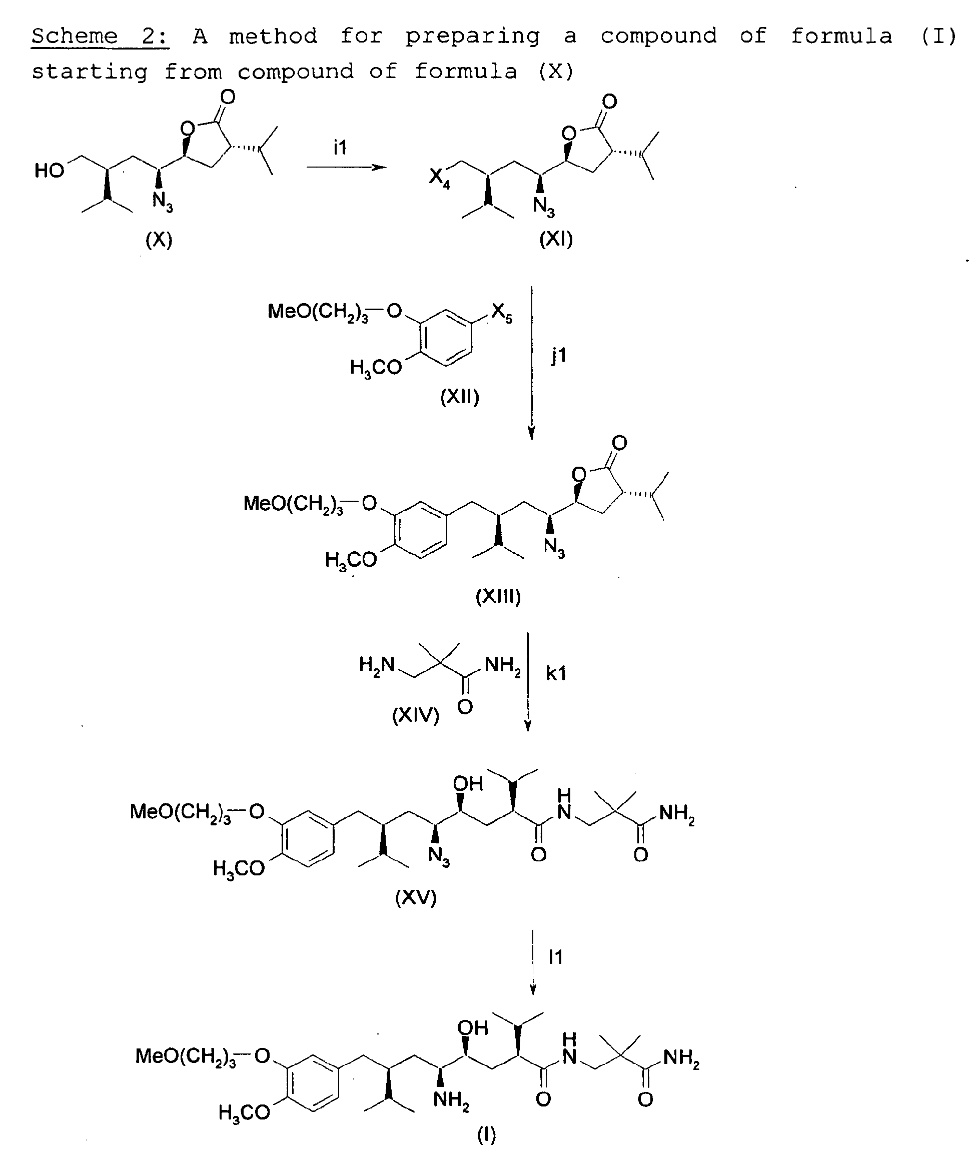

In an alternative option of the invention, the process for preparing aliskiren can be characterized by steps (a) to (h) as shown above in Scheme 1 and subsequent steps (i2) to (m2) as depicted in scheme 3 below.

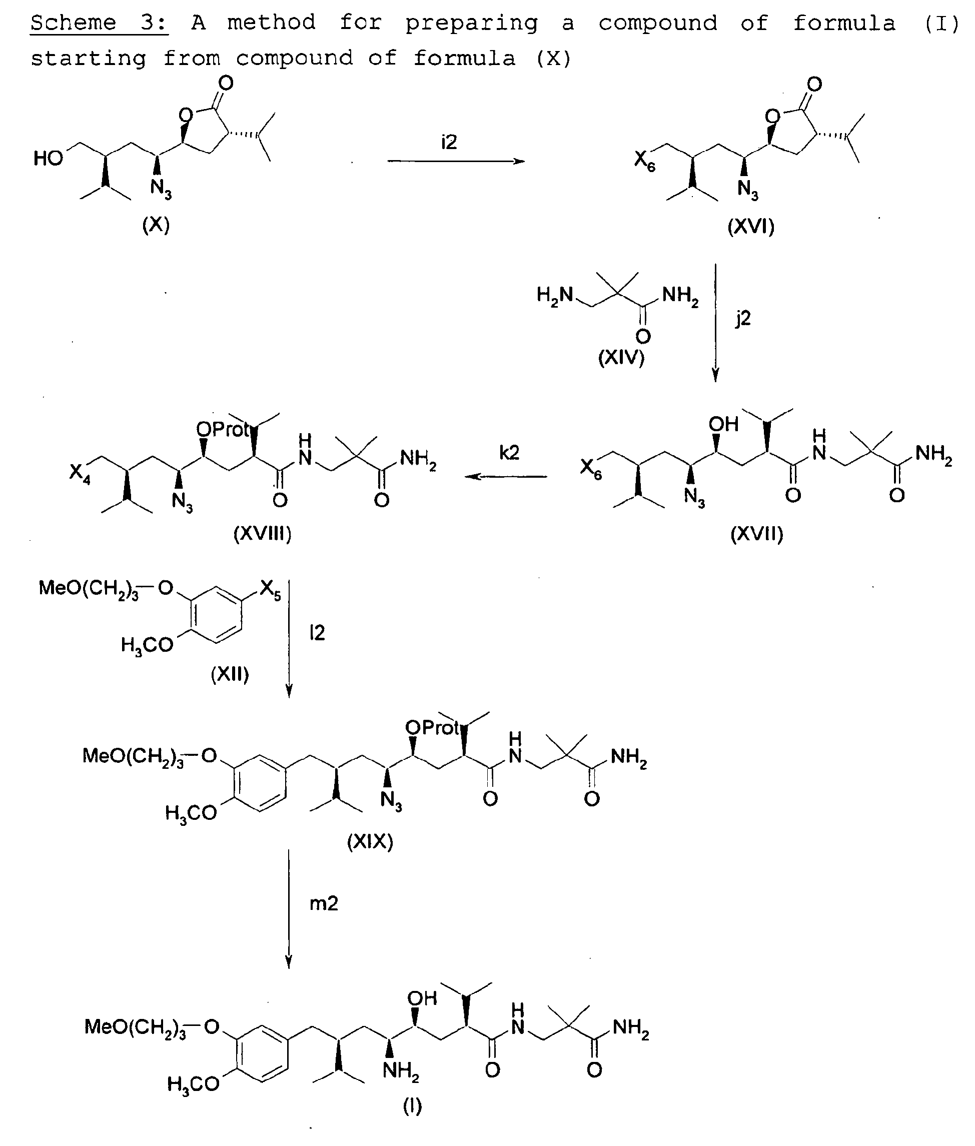

The compound of formula (I) prepared by the process according to the invention is aliskiren in the form of its free base or a pharmaceutically acceptable salt thereof. Examples of suitable salts include addition salts with inorganic acids such as hydrochloride acid, hydrobromic acid, phosphoric acid, sulfuric acid and nitric acid, or organic acids such as carboxylic acids, e.g. fumaric acid, acetic acid, citric acid, tartaric acid, maleic acid and glutamic acid, or sulfonic acids, e.g. methanesulfonic acid. Aliskiren or the pharmaceutically acceptable salts thereof can be prepared in a non-crystalline, i.e. amorphous, form or a crystalline form including any polymorphic or pseudopolymorphic forms such as solvates like hydrates. Most preferably, the compound of formula (I) is prepared in the form of its hemifumarate salt.

Preferably, the obtained aliskiren or salt thereof has a purity of at least 99.5 %.

In addition to the compound of formula (I), also compounds of formulae (II) to (XIX) can not only be present and used in the form as is shown by formulae (II) to (XIX), but can also be present and used in the form of a salt thereof, e.g. a salt formed by an organic or inorganic base and an acidic part of the compound such as an alcohol group or a salt formed by an organic or inorganic acid and a basic part of the compound such as an amino group. Moreover, compounds (II) to (XIX) can also be present and used in the form of a solvate thereof such as a solvate formed by a solvent used for the preparation of a compound of formulae (II) to (XIX). Thus, whenever a compound is referred to in this description or the appended claims, a corresponding salt or solvate is also intended, provided such is possible or appropriate under the circumstances.

Furthermore, the compounds (II) to (XIX) used in the process according to the invention can comprise protecting groups.

Protecting groups may be introduced to protect the functional groups present from undesired reaction with reaction components under the conditions used for carrying out a particular chemical transformation of the present invention. The need and choice of protecting groups for a particular reaction is known to those skilled in the art and depends on the nature of the functional group to be protected (for example, amino, hydroxyl, thiol etc.), the structure and stability of the molecule of which the substituent is a part and the reaction conditions. Well-known protecting groups which meet these conditions as well as their introduction and removal are described, for example, in Kocienski, "Protecting Groups", Georg Thieme Verlag, Stuttgart, (2005); Greene and Wuts, "Protective Groups in Organic Synthesis", John Wiley & Sons, NY (1999).

The individual reaction steps used in the process according to the invention can generally be carried out according to standard methods, in the presence or absence of a diluent or solvent, which is preferably inert to the reagents, of catalysts and/or inert atmospheres. The temperature used in the reaction steps of the process according to the invention can generally range from low temperatures such as -78°C to room temperature or elevated temperatures, which are preferably at or near the boiling point of the solvent used. In addition, the individual reaction steps are generally carried out at atmospheric or super-atmospheric pressure.

Preferably, the individual reaction steps of the process according to the invention are carried out in the presence of a solvent. Suitable solvents are water, organic solvents and ionic liquids, especially polar organic solvents or mixtures of at least two solvents. In particular, examples of suitable solvents include hydrocarbons such as petroleum ether, pentane, hexane, heptane, cyclohexane, methylcyclohexane, benzene, toluene, xylene; halogenated hydrocarbons such as dichloromethane, chloroform, tetrachloroethane, chlorobenzene; ethers such as diethyl ether, dibutyl ether, tetrahydrofuran, 2-methyltetrahydrofuran, dioxane, ethylene glycol dimethyl, diethyl ether; carbonic esters and lactones such as methyl acetate, ethyl acetate, isopropyl acetate, methyl propionate, valerolactone; N,N-substituted carboxamides and lactams such as dimethylformamide, dimethylacetamide, N-methylpyrrolidinone; ketones such as acetone, methylisobutylketone, cyclohexanone; sulfoxides and sulfones such as dimethysulfoxide, dimethylsulfone, tetramethylene sulfone; alcohols such as methanol, ethanol, n- or i-propanol, n-, i- or t-butanol, pentanol, hexanol, cyclohexanol, cyclohexanediol, benzyl alcohol, ethylene glycol, diethylene glycol, propanediol, butanediol, ethylene glycol momomethyl or monoethyl ether, diethylene glycol monomethyl or monoethyl ether; nitriles such as acetonitrile, propionitrile; tertiary amines such as trimethylamine, triethylamine, tripropylamine, tributylamine, pyridine, N-methylpiperazine, N-methylmorpholine; organic acids such as acetic acid, formic acid; ionic liquids; and mixtures thereof as well as mixtures of at least one of the abovementioned solvents with water.

Moreover, the individual reaction steps of the process according to the invention are preferably carried out under an inert atmosphere, more preferably under nitrogen atmosphere.

The compounds of formulae (I) to (XIX) may be isolated using conventional methods known in the art, e.g. extraction, distillation, crystallization and filtration as well as combinations thereof.

In the following, preferred embodiments of substituents X₁ to X₆ and Prot are given. These preferred embodiments can independently be combined with each other.

X₁ is an alkylaryl group, i.e. an alkyl group which is substituted with an aryl group. In particular, X₁ is -(CH₂)ₙ-aryl, wherein n is preferably ranging from 1 to 6 and aryl is preferably phenyl or naphthyl. Most preferably, X₁ is benzyl, i.e. -CH₂C₆H₅.

Preferably, X₂ is selected from the group consisting of Br, Cl and I. Most preferably, X₂ is Br.

Preferably, X₃ is selected from the group consisting of Br, Cl and I. Most preferably, X₃ is Br.

Preferably, X₄ is selected from the group consisting of Br, Cl and I. Most preferably, X₄ is Br.

Preferably, X₅ is selected from the group consisting of Br, Cl and I. Most preferably, X₅ is Br.

Preferred examples of X₆ include tert.-butyldimethylsilyl, trimethylsilyl, triethylsilyl, triisopropylsilyl and tert.-butyldiphenylsilyl. Most preferably, X₆ is tert.-butyldimethylsilyl.

Preferably, the protecting group Prot of compounds (XVIII) and (XIX) is an unsubstituted or substituted benzyl group. Most preferably, Prot is -CH₂C₆H₅.

In the following, preferred embodiments of the individual reaction steps are given. These preferred embodiments can independently be combined with each other.

### Step (a):

Isovaleric acid having formula (II) is preferably reacted with a (S)-4-X₁-oxazolidin-2-one such as (S)-4-benzyloxazolidin-2-one to yield a compound of formula (III). It is particularly preferred that the isovaleric acid (II) is firstly converted into isovaleric acid halogenide and said isovaleric acid halogenide is then reacted with an (S)-4-X₁-oxazolidone-2-one anion such as (S)-4-benzyloxazolidin-2-one anion. The (S)-4-X₁₋ oxazolidone-2-one anion can be obtained by treating (S)-4-X₁-oxazolidone-2-one with a suitable base.

The generation of isovaleric acid halogenide such as isovaleric chloride can be performed with oxalyl chloride alone or in the presence of a base such as pyridine, in a solvent such as dimethylformamide. Further, isovaleric acid can be deprotonated with a base such as lithium hydroxide followed by treatment of the obtained carboxylate with trimethysilylchloride and oxalyl chloride. Alternatively, the generation of isovaleric acid halogenide such as isovaleric chloride can be performed using thionyl chloride alone or in the presence of a base such as lithium hydroxide, in anhydrous solvents such as toluene or benzene. Most preferably, the isovaleric acid halogenide is generated by treatment of isovaleric acid with oxalyl chloride in toluene at a temperature of 5°C to 65°C using dimethylformamide (Tetrahedron 1997, 53, 13757).

The (S)-4-X₁-oxazolidin-2-one anion can be generated by treating (S)-4-X₁-oxazolidin-2-one with a suitable base such as n-butyl litium in a solvent such as tetrahydrofuran or dimethylformamide. The temperature for this reaction is preferably about -78°C. Preferably, the obtained (S)-4-X₁-oxazolidin-2-one anion such as (S)-4-benzyloxazolidin-2-one anion is reacted with the isovaleric acid halogenide such as isovaleric chloride in a solvent such as tetrahydrofuran or dimethylformamide.

### Step (b) :

The compound of formula (III) is preferably reacted with allyl bromide to yield a compound of formula (IV). It is particularly preferred that the compound of formula (III) is condensed with allyl bromide in the presence of a suitable base and an inert organic solvent. As illustrated in Scheme 1, chirality is introduced via Evans technology by employing an (S)-oxazolidinone derivative. Examples for suitable bases employed in the condensation include, but are not limited to, n-butyl lithium, lithium diisopropylamide (LDA), lithium hexamethyldisilazide, sodium hexamethyldisilazide and potassium hexamethyldisilazide. Commonly used solvents are for example tetrahydrofuran, dichloromethane, toluene or mixtures thereof. Step (b) is preferably performed at a temperature ranging from about -78°C to room temperature. Most preferably, the compound of formula (III) is reacted with allyl bromide in tetrahydrofuran using lithium hexamethyl disilazide at a temperature of -70°C.

### Step (c):

The compound of formula (IV) is converted to a compound of formula (V) by removal of the oxazolidinone group. Removal of the oxazolidinone group can generally be carried out according to methods well known in the art, e.g., using lithium peroxide in an organic solvent such as THF. Most preferably, step (c) is carried out in a 3:1 (v/v) mixture of tetrahydrofuran and water at a temperature of 0°C using a mixture of LiOH and 30% H₂O₂

### Step (d):

The compound of formula (V) is preferably heated in the presence of acetic anhydride and/or P₂O₅ to a temperature ranging from room temperature to the boiling temperature of the reaction mixture.

### Step (e):

The compound of formula (VI) is preferably converted into a compound of formula (VII) in the presence of Grubbs catalyst such as Cl₂(P(cymene)₃)₂Ru=CHPh. Solvents suitable for this reaction include chlorinated hydrocarbons such as methylene chloride and the like, or aromatic hydrocarbons such as toluene and the like, or ionic liquids. Preferably, in step (e) the reaction mixture is heated to a temperature ranging from room temperature to the boiling temperature of the reaction mixture. Most preferably, the compound of formula (VI) is converted into the compound of formula (VII) in the presence of Cl₂(P(cymene)₃)₂Ru=CHPh using dichloromethane at reflux temperature.

### Step (f):

Preferably, in step (f) the compound of formula (VII) is reduced by a reducing agent, preferably HOAc in the presence of Zn (Zn/HOAc), hydrogen in the presence of Pt (H₂/Pt), RuCl₂(PhP)₃, NaBH₄ or LiAlH₄, and then hydrolyzed to a compound of formula (VIII) or (VIIIa). Suitable solvents for the reduction step include for example tetrahydrofurane and dioxane. The hydrolysis of the reduced ester intermediate to a compound of formula (VIII) or (VIIIa) can conveniently be carried out by means of sodium hydroxide in the presence of an suitable solvent such as water followed by the use of an aqueous solution of hydrochloric acid. Most preferably, the compound of formula (II) is reduced by LiAlH₄ using tetrahydrofuran and then hydrolyzed using NaOH in water followed by using HCl in water.

### Step (g):

Preferably, in step (g) the compound of formula (VIII) or (VIIIa) is lactonized in the presence of a halogenation agent selected from the group consisting of Br₂, I₂, N-halogenated carboxamides, N-halogenated dicarboxamides, N-halogenated phthalimides, N-halogenated succinimides, N-halogenated sulfonamides, N-halogenated imides, hypochlorites and mixtures thereof. In particular, suitable halogenation agents include, but are not limited to, elemental bromine and iodine, in particular N-chloro, N-bromo and N-iodocarboxamides and dicarboximides. Preferred are N-chloro, N-bromo and N-iodophthalimide and especially N-chloro, N-bromo and N-iodosuccinimide, as well as tertiary butyl hypochlorite and N-halogenated sulfonamides and imides, for example chloroamine T.

It is preferred to carry out step (g) in organic solvents, more preferably water-miscible solvents, for example tetrahydrofuran or dioxane. The reaction temperature can range from about -70°C to ambient temperature.

Furthermore, step (g) is advantageously carried out in the presence of at least one inorganic or organic acid and at least one equimolar amount of water, based on the compound of formula (VIII) or (VIIIa). Examples of suitable acids include formic acid, acetic acid, methanesulfonic acid, trifluoroacetic acid, trifluoromethanesulfonic acid, toluenesulfonic acid, H₂SO₄, H₃PO₄, HCl and HBr.

Most preferably, step (g) is carried out using N-bromosuccinimide in the presence of H₃PO₄ and a mixture of tetrahydrofuran and water as a solvent.

### Step (h):

Preferably, step (h) is carried out in a mixture of an organic solvent and water in the presence of an azidation agent. In particular, the reaction is carried out in water-miscible organic solvents mixed with water, typically for example alcohols or ethers such as methanol, ethanol, ethylene glycol, diethylene glycol, diethylene glycol monomethyl or ethyl ether, diethyl ether, tetrahydrofuran or dioxane. Examples of suitable azidation agents include metal azides, especially alkaline earth metal azides and alkali metal azides, as well as silyl azides. Especially preferred azidation agents are lithium azide, sodium azide and potassium azide. Moreover, it is preferred that step (h) is carried out in the presence of a phase transfer catalyst.

Most preferably, step (h) is carried out in tripropylene glycol in the presence of NaN₃.

### Step (i1):

The compound of formula (X) is preferably converted into a compound of formula (XI) in the presence of a suitable acid halogenide, such as acid chlorides and acid bromides, or PBr₃ in a polar organic solvent such as dichloromethane at a temperature of -20 to 50°C.

### Step (j1) or (12):

Generally, the coupling of Grignard reagents with alkenyl halides in an ether such as, for example, tetrahydrofuran or dioxan as solvents in the presence of catalytic quantities of a soluble metal complex, for example an iron complex such as iron acetonyl acetate, and in the presence of more than equimolar quantities of a solvent stabilizing the metal complex, for example N-methylpyrrolidone, is described by G. Cahiez et al. in Synthesis (1998), pages 1199-1200.

Preferably, the metal catalyst used in step (j1) or step (12) is iron acetonyl acetate. It is particularly preferred that the metal catalyst is present in catalytic amounts, i.e. 0.1 to 20 wt.-%, based on a compound of formula (XI) or (XVIII).

Further, it is preferred that step (j1) or step (12) is/are carried out in the presence of a solvent stabilizing the complex formed by the metal catalyst. Preferably, said solvent is N-methylpyrrolidone.

Advantageously, step (j1) or step (12) is carried out at a temperature of 50°C to 80°C, more preferably 40 to 70°C.

In step (j1) the compound of formula (XII) can firstly be reacted with a metal, preferably an earth alkaline metal such as magnesium, and the obtained Grignard product is then reacted with the compound of formula (XI) in the presence of said metal catalyst. Alternatively, in step (j1) the compound of formula (XI) is firstly reacted with a metal, preferably an earth alkaline metal such as magnesium, and the obtained Grignard product is then reacted with the compound of formula (XII) in the presence of said metal catalyst.

Likewise, in step (12) the compound of formula (XII) can firstly be reacted with a metal, preferably an earth alkaline metal such as magnesium, and the obtained Grignard product is then reacted with the compound of formula (XVIII) in the presence of said metal catalyst. Alternatively, in step (12) the compound of formula (XVIII) is firstly reacted with a metal, preferably an earth alkaline metal such as magnesium, and the obtained Grignard product is then reacted with the compound of formula (XII) in the presence of said metal catalyst.

Preferably, step (j1) is carried out by firstly reacting a compound of formula (XI) or (XII) with magnesium using 1,2-dibromoethane and tetrahydrofuran at reflux temperature. Subsequently, the obtained product is reacted with a compound of formula (XII) or (XI) in the presence of Fe(acac)₃ using tetrahydrofuran and N-methylpyrrolidone.

Preferably, step (12) is carried out by firstly reacting a compound of formula (XVIII) or (XII) with magnesium using 1,2-dibromoethane and tetrahydrofuran at reflux temperature. Subsequently, the obtained product is reacted with a compound of formula (XII) or (XVIII) in the presence of Fe(acac)₃ using tetrahydrofuran and N-methylpyrrolidone.

### Step (k1):

The reaction of a compound of formula (XIII) with a compound of formula (XIV) by opening of the lactone ring is usually carried out in the presence of alcohols or amines which are capable of forming activated carboxylic acid esters or carboxamides. Such compounds are well-known. Examples therefor include 2-hydroxypyridine, N-hydroxycarboxamides and imides; and carboximides such as N-hydroxysuccinimide. Organic solvents are used as solvent, with tertiary amines being of advantage, for example trimethyl or triethyl amines. The reaction temperature may range for example from about 40°C to 150°C.

Most preferably, step (k1) is carried out in the presence of 2-hydroxypyridine using triethyl amine at a temperature of about 80°C.

### Step (11):

The reduction of the azide group of the compound of formula (XV) to an amine group takes place in a manner known per se, for example using metal hydrides or more preferably using a catalytic method with hydrogen in the presence of homogeneous (Wilkinson catalyst) or heterogeneous catalysts, for example Raney nickel or precious metal catalysts such as platinum or palladium, if necessary on substrates such as carbon. The hydrogenation can also be carried out if necessary catalytically under phase transfer conditions, for example with ammonium formate as hydrogen donor. It is of advantage to use organic solvents. The reaction temperature may range for example from approximately 0° C. to 200° C. Hydrogenation may be carried out at normal pressure or increased pressure up to 100 bar. Most preferably, step (11) is carried out at room temperature and 4 bar of hydrogen using a Pd/C catalyst, ethanolamine and methanol.

### Step (i2):

Preferably, in step (i2) the compound of formula (X) is reacted with an appropriate silylhalogenide such as trialkylsilylchloride. In particular, the compound of formula (X) is reacted with a compound selected from the group consisting of tert.-butyldimethylsilylchloride, trimethylsilylchloride, triethylsilylchloride, triisopropylsilylchloride and tert.-butyldiphenylsilylchloride. Advantageously, the solvents used for this reaction are chlorinated hydrocarbons such as methylene chloride and the like. The base used for this reaction can be a tertiary amine such as N-methyl imidazole.

### Step (j2) :

Generally, the preferred embodiments for step (j2) correspond to those of step (k1).

Thus, step (j2) is preferably carried out in the presence of a compound selected from the group consisting of 2-hydroxypyridine, N-hydroxycarboxamides, N-hydroxycarboximides such as N-hydroxysuccinimide, and mixtures thereof.

Further, it is preferred that in step (j2) a tertiary amine such as trimethyl amine and/or triethyl amine is used as a solvent.

Most preferably, step (j2) is carried out in the presence of 2-hydroxypyridine using triethyl amine at a temperature of about 80°C.

### Step (k2):

Preferably, in step (k2) firstly the hydroxy group of the compound of formula (XVII) is protected by a protecting group Prot. Methods for introducing the protection group Prot are known to those skilled in the art. Preferably, the compound of formula (XVII) is reacted with benzylbromide. Secondly, substituent X₆ is replaced by substituent X₄. This can for example be effected by reacting the obtained protected intermediate with an halogenating agent such as PBr₃ in the presence of tetrabutyl ammonium fluoride.

### Step (m2):

Generally, the preferred embodiments for step (m2) correspond to those of step (11).

Thus, step (m2) is most preferably be carried out at room temperature and 4 bar of hydrogen using a Pd/C catalyst, ethanolamine and methanol.

As mentioned above, the present invention also includes any variant of the above process, in which the reaction components are used in the form of their salts or solvates.

The present invention further includes any variant of the above process, in which an intermediate product obtainable at any stage is used as the starting material, and the remaining steps described above or other steps are carried out to arrive at aliskiren of formula (I) or a pharmaceutical acceptable salt thereof.

Thus, the invention in particular relates to a process for preparing aliskiren or a pharmaceutically acceptable salt thereof comprising at least one reaction step selected from the group consisting of step (a), i.e. converting compound (II) into compound (III), step (b), i.e. converting compound (III) into compound (IV), step (c), i.e. converting compound (IV) into compound (V), step (d), i.e. converting compound (V) into compound (VI), step (e), i.e. converting compound (VI) into compound (VII), step (f), i.e. converting compound (VII) into compound (VIII) or (VIIIa), step (g), i.e. converting compound (VIII) or (VIIIa) into compound (IX), step (h), i.e. converting compound (IX) into compound (X), step (i1), i.e. converting compound (X) into compound (XI), step (j1), i.e. converting compound (XI) into compound (XIII), step (i2), i.e. converting compound (X) into compound (XVI), step (j2), i.e. converting compound (XVI) into compound (XVII), step (k2), i.e. converting compound (XVII) into compound (XVIII), step (12), i.e. converting compound (XVIII) into compound (XIX), and combinations of these reaction steps.

The compounds of formulae (VI), (VII), (VIII), (IX), (X), (XI), (XVI), (XVII) and (XVIII) as well as their use for the manufacture of aliskiren are further objects of the present invention.

In addition, the invention is directed to a process for preparing aliskiren or a pharmaceutically acceptable salt thereof using at least one compound of formulae (VI), (VII), (VIII), (IX), (X), (XI), (XVI), (XVII) and (XVIII).

Further, the invention is also directed to a process for preparing a pharmaceutical composition containing aliskiren or a pharmaceutically acceptable salt thereof which process comprises preparing aliskiren or a pharmaceutically acceptable salt thereof in accordance with a process according to the invention described above and converting the aliskiren or salt thereof into a pharmaceutical composition. Formulations of aliskiren are known from e.g. EP-A-0 678 503, EP-A-1 517 682 and WO-A-2005/089729. Preferably, the conversion of aliskiren or pharmaceutically acceptable salt thereof into a pharmaceutical composition is effected by a hot-melt and solvent-free granulation process. Hot-melt granulation of aliskiren is for example described in co-pending application EP 07 006 055.3.

Aliskiren or pharmaceutically acceptable salts thereof can also be used in pharmaceutical compositions that exhibit synergistic effects of aliskiren in combination with ACE inhibitors, ARBs, antidiabetics or diuretics which have been previously described in e.g. EP-A-1 341 533, EP-A-1 602 370, EP-A-1 507 558, US-A-2003/0114389, WO-A-03/099279, WO-A-2005/070406, and WO-A-2005/077418.

The invention is further illustrated with reference to the following examples and figures.
Figure 1 is an X-ray powder diffractogram of aliskiren hemifumarate obtained according to Example 1.
Figure 2 is an FT-IR spectrum of aliskiren hemifumarate obtained according to Example 1.
Figure 3 is a DSC thermogram of alsiskiren hemifumarate obtained according to Example 1.
Figures 4 and 5 are images of aliskiren hemifumarate obtained according to Example 1 which were recorded using optical microscopy.
Figure 6 is an X-ray powder diffractogram of aliskiren hemifumarate obtained according to Example 2.
Figure 7 is a DSC thermogram of aliskiren hemifumarate obtained according to Example 2.

### Examples

### Example 1: Preparation of aliskiren hemifumarate

9 g of aliskiren were dissolved in 26 ml of ethanol and mixed with 0.95 g of fumaric acid. The mixture was filtered off and the solvent was evaporated. The residue was dissolved in 220 ml of acetonitrile and stirred at ambient temperature. The resulting solution was seeded with 50 mg of aliskiren hemifumarate and agitated at ambient temperature for 17 hours. The suspension was cooled to -10°C and filtered off by suction after 1 hour. The residue was washed with 3 x 25 ml of acetonitrile and then dried in a vacuum oven at 30°C for 1 hour. The aliskiren hemifumarate was obtained as white crystals (7.6 g).

¹H-NMR (DMSO-d6) δ/ppm: 7.62 (t, 1H), 7.20 (s, 1H), 6.83-6.79 (m, 3H), 6.69 (dd, 1H), 6.35 (s, 1H), 3.97 (t, 2H), 3.71 (s, 3H), 3.46 (t, 2H), 3.28 (m, 1H), 3.23 (s, 3H), 3.18-3.08 (m, 2H), 2.57 (m, 1H), 2.46-2.25 (m, 3H), 1.92 (m, 2H), 1.79 (m, 1H), 1.69-1.52 (m, 3H), 1.41-1.21 (m, 3H), 1.03 (s, 6H), 0.85-0.76 (m, 12H).

¹³C-NMR (DMSO-δ6) d/ppm: 178.5, 174.7, 170.2, 147.8, 147.2, 135.9, 133.4, 121.1, 114.2, 112.0, 69.3, 68.7, 65.4, 57.9, 55.5, 54.2, 48.9, 46.3, 42.6, 40.2, 36.6, 33.9, 31.7, 30.4, 29.2, 28.3, 23.5, 20.7, 20.0, 19.4, 17.1.

MS (Q-TOF): m/z 552.4 (MH+).

### HRMS (Q-TOF): m/z 552.4017 (calculated (C30H54N3O6): 552.4013)

### X-ray powder diffraction:

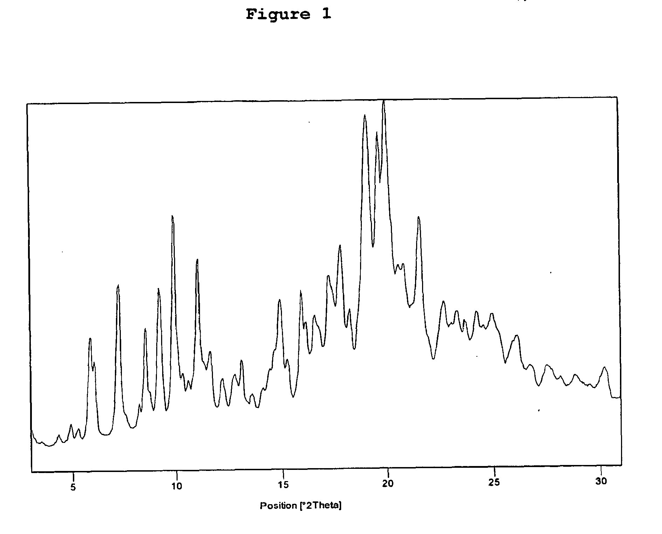

A sample of the obtained aliskiren hemifumarate was measured by X-ray powder diffractometry. The XRD pattern was obtained on a Philips PW3040/60 X'Pert powder diffractometer using X'celerator detector at CuKα radiation, 1.54178 Å, 3°<2θ<30°. The obtained X-ray powder diffractogram is shown in Figure 1 and is characterized by the following peaks:

| | Pos. | d-spacing | Rel. Int. |
|---|---|---|---|
| No. | [°2Th.] | [A] | [%] |
| 1 | 7.3 | 12.07 | 47 |
| 2 | 10.0 | 8.89 | 67 |
| 3 | 11.0 | 8.01 | 55 |
| 4 | 14.9 | 5.93 | 41 |
| 5 | 17.8 | 4.97 | 58 |
| 6 | 19.1 | 4.65 | 95 |
| 7 | 20.0 | 4.44 | 100 |
| 8 | 21.6 | 4.11 | 66 |

### FT-IR spectroscopy:

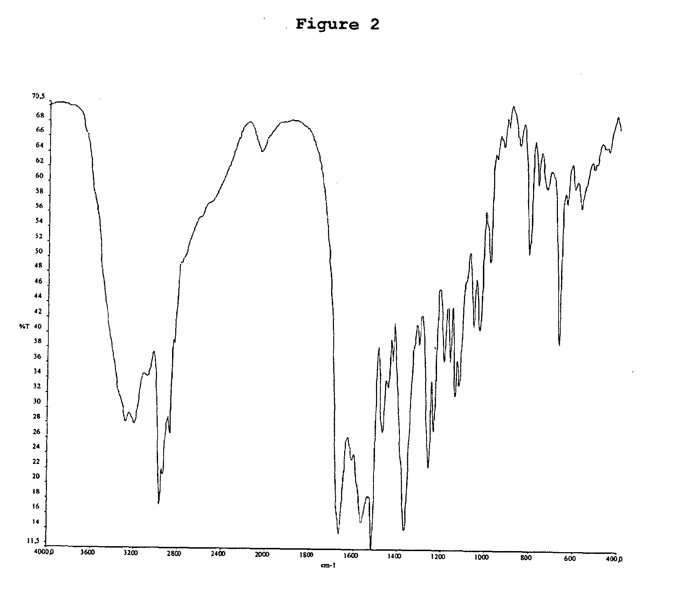

FT-IR spectra of KBr discs prepared from the obtained aliskiren hemifumarate were recorded over a wave number range of 4000-400 cm⁻¹ on a Perkin Elmer FT-IR spectrometer Spectrum GX at a resolution of 4 cm⁻¹. The obtained FT-IR spectrum is shown in Figure 2.

### Thermal analysis:

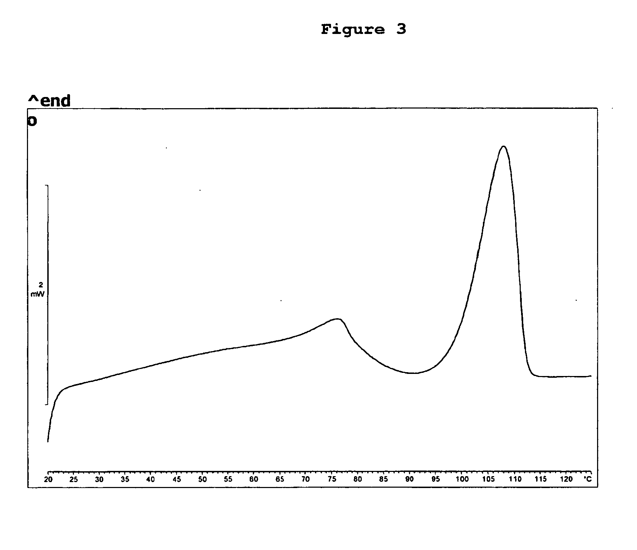

DSC thermograms were recorded on a DSC 822e Mettler Toledo scanning calorimeter. Samples of approx. 3 mg were scanned between 20°C and 200°C at a heating rate of 10°C/min under nitrogen atmosphere. The obtained DSC thermogram is shown in Figure 3.

### Microscopy:

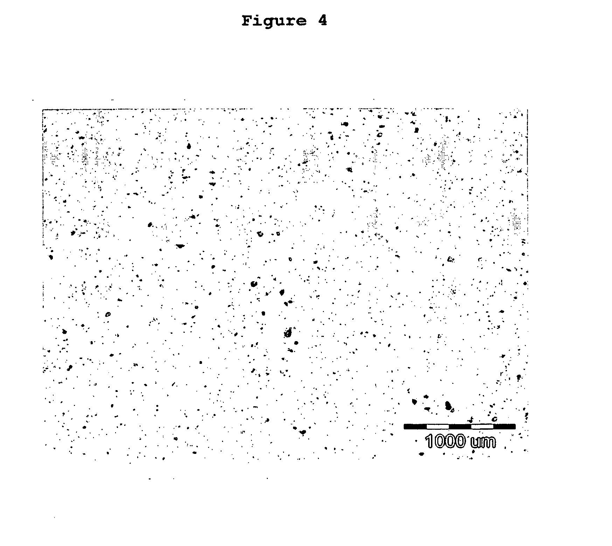

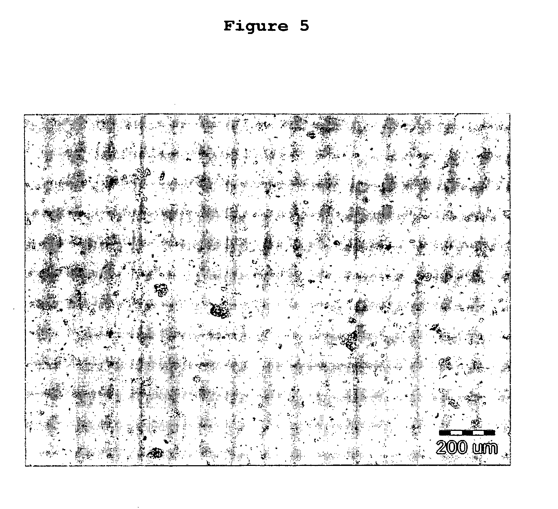

Images of particles of the obtained aliskiren hemifumarate were taken on a Microscope Olympus BX 50 equipped with Olympus camera DP70. The sample was prepared as suspension of aliskiren hemifumarate in paraffinic oil. The obtained images are shown in Figures 4 and 5.

### Example 2: Preparation of amorphous aliskiren hemifumarate

Aliskiren hemifumarate obtained according to Example 1 was milled using a ball mill at 20 Hz for 30 min.

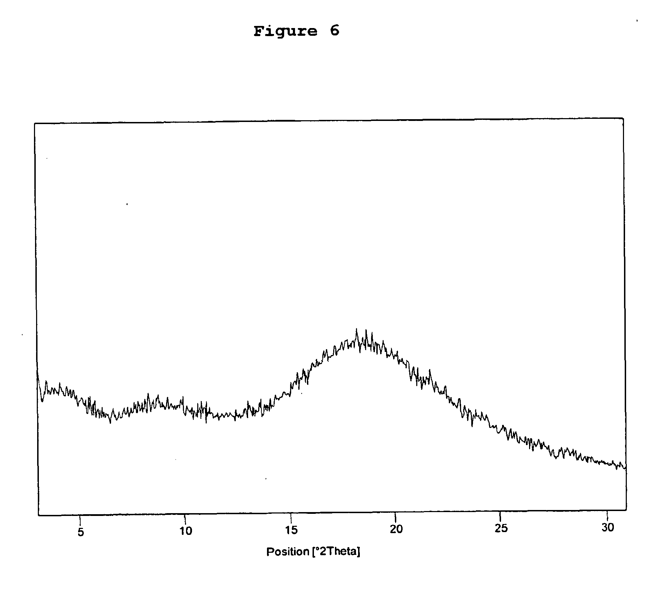

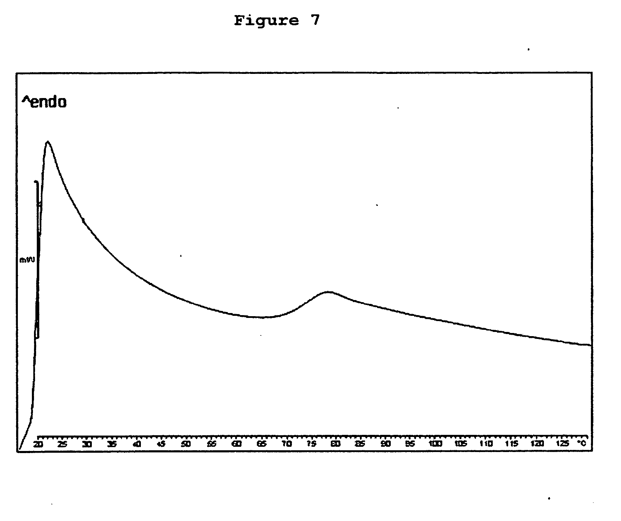

An X-ray powder diffractogram of the obtained amorphous aliskiren hemifumarate is shown in Figure 6. Moreover, the corresponding DSC thermogram is shown in Figure 7.

## Claims

1. Process for preparing aliskiren of the following formula (I) or a pharmaceutically acceptable salt thereof 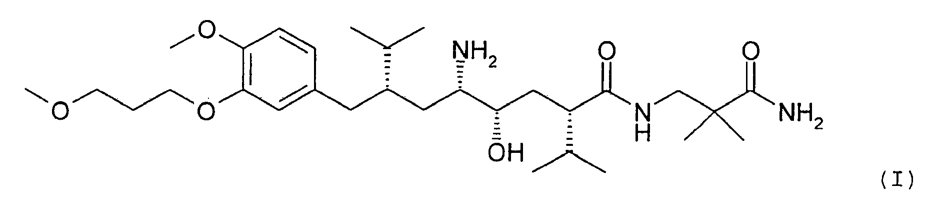 comprising
(a) converting a compound of formula (II) 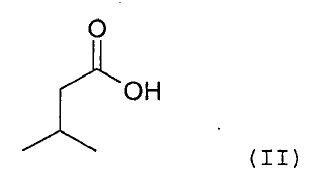 into a compound of formula (III) 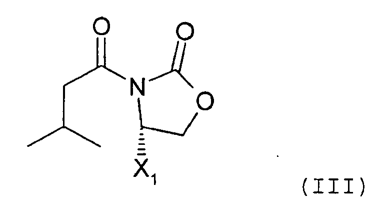 wherein X₁ is an alkylaryl group,
(b) reacting the compound of formula (III) with 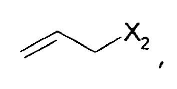 wherein X₂ is halogen, to form a compound of formula (IV) 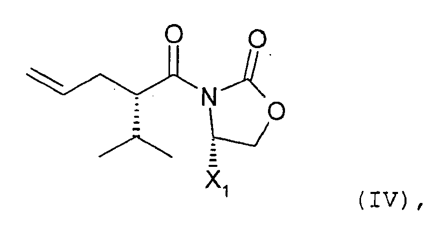
(c) converting the compound of formula (IV) into a compound of formula (V) 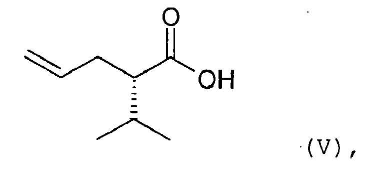
(d) converting the compound of formula (V) into a compound of formula (VI) 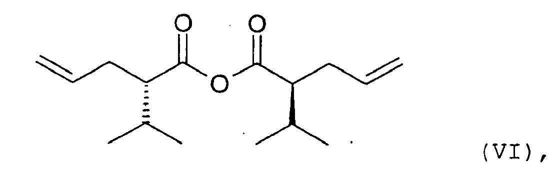
(e) cyclizing the compound of formula (VI) in the presence of a metal catalyst to form a compound of formula (VII) 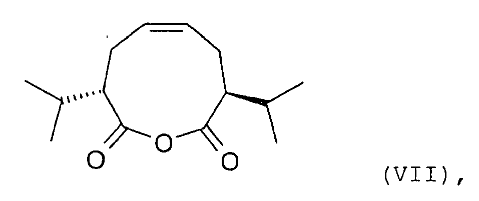
(f) converting the compound of formula (VII) into a compound of formula (VIII) or (VIIIa) 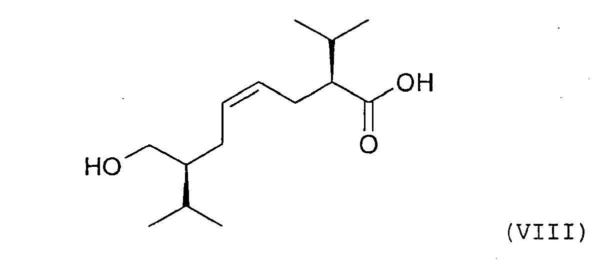 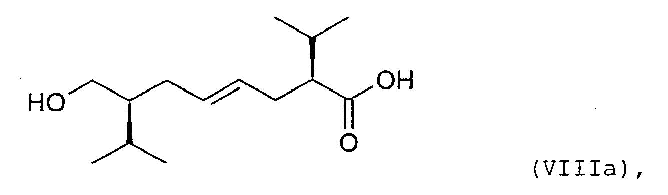
(g) lactonizing the compound of formula (VIII) or (VIIIa) into a compound of formula (IX) 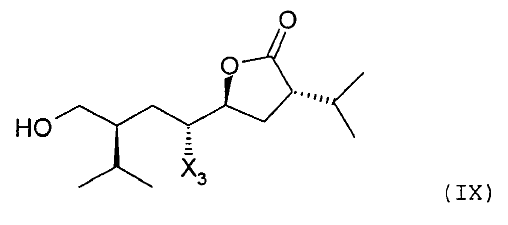 wherein X₃ is halogen,
(h) azidating the compound of formula (IX) to form a compound of formula (X) 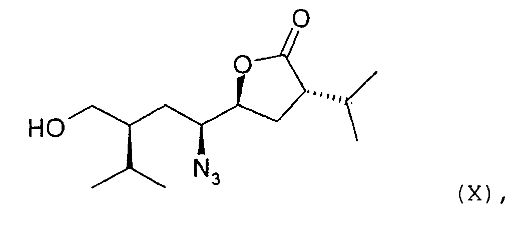 and either
(i1) converting the compound of formula (X) into a compound of formula (XI) 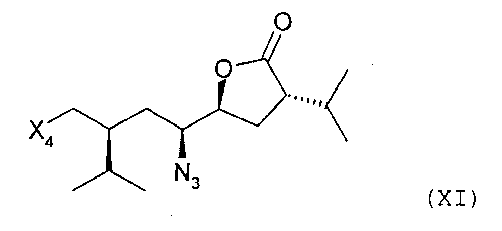 wherein X₄ is halogen,
(j1) reacting the compound of formula (XI) with a compound of formula (XII) in the presence of a metal catalyst 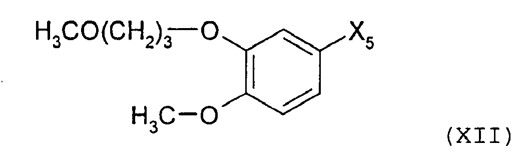 wherein X₅ is halogen,
to form a compound of formula (XIII) 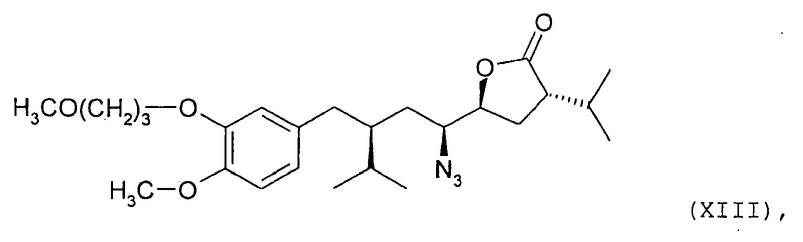
(k1) reacting the compound of formula (XIII) with an amine of formula (XIV) 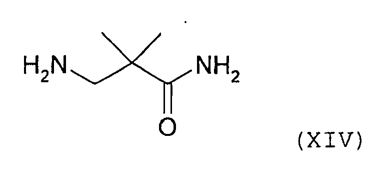 to form a compound of formula (XV) 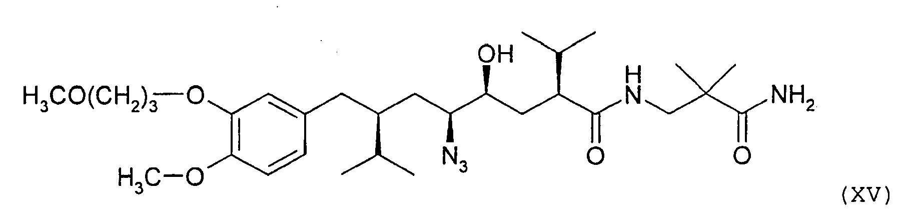 and
(l1) converting the compound of formula (XV) into the compound of formula (I) or a pharmaceutically acceptable salt thereof,
or
(i2) converting the compound of formula (X) into a compound of formula (XVI) 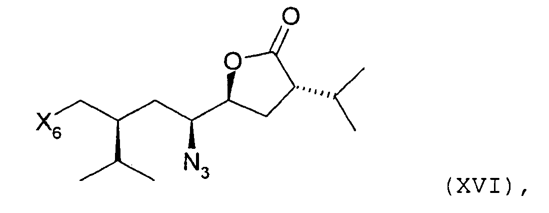 wherein X₆ is selected from the group consisting of trialkylsilyl, dialkylarylsilyl, alkyldiarylsilyl and triarylsilyl,
(j2) reacting the compound of formula (XVI) with an amine of formula (XIV) 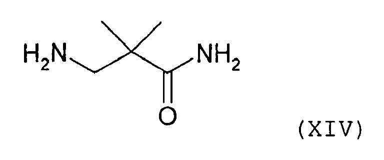 to form a compound of formula (XVII) 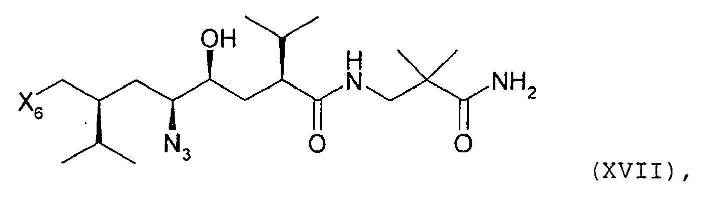
(k2) converting the compound of formula (XVII) into a compound of formula (XVIII) 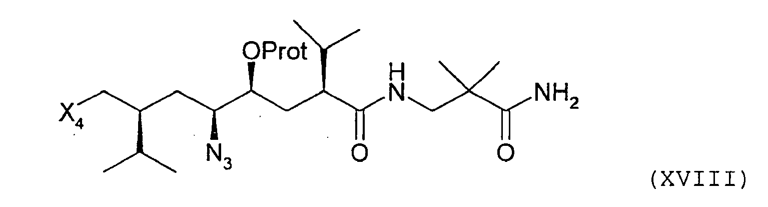 wherein X₄ is halogen and Prot is a hydroxyl protecting group,
(l2) reacting the compound of formula (XVIII) with a compound of formula (XII) in the presence of a metal catalyst 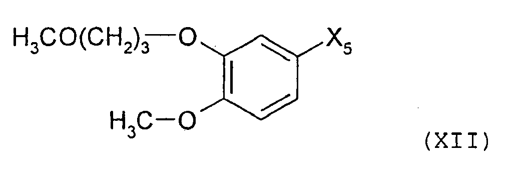 wherein X₅ is halogen,
to form a compound of formula (XIX) 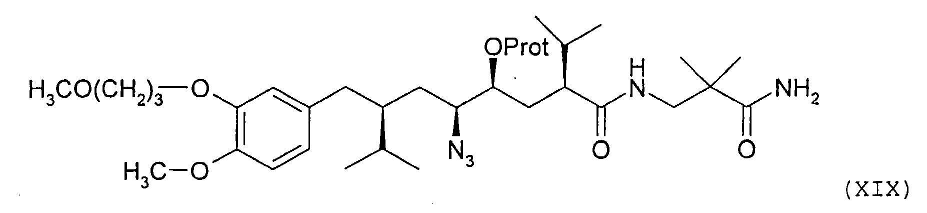 and
(m2) converting the compound of formula (XIX) into the compound of formula (I) or a pharmaceutically acceptable salt thereof.

2. Process according to claim 1, wherein in step (a) the compound of formula (II) is converted into isovaleric acid halogenide and said isovaleric acid halogenide is reacted with an (S)-4-X₁-oxazolidone-2-one anion.

3. Process according to claim 1 or 2, wherein in step (d) the compound of formula (V) is heated in the presence of acetic anhydride and/or P₂O₅ to a temperature ranging from room temperature to the boiling temperature of the reaction mixture.

4. Process according to any one of claims 1 to 3, wherein in step (e) the metal catalyst is a Grubbs catalyst, such as Cl₂(P(cymene)₃)₂Ru=CHPh, and the reaction mixture is heated to a temperature ranging from room temperature to the boiling temperature of the reaction mixture.

5. Process according to any one of claims 1 to 4, wherein in step (f) the compound of formula (VII) is reduced by a reducing agent, preferably Zn/HOAc, H₂/Pt, RuCl₂(PhP)₃, NaBH₄ or LiAlH₄, and then hydrolyzed to a compound of formula (VIII) or (VIIIa).

6. Process according to any one of claims 1 to 5, wherein in step (g) the compound of formula (VIII) or (VIIIa) is lactonized in the presence of a halogenation agent selected from the group consisting of Br₂, I₂, N-halogenated carboxamides, N-halogenated dicarboxamides, N-halogenated phthalimides, N-halogenated succinimides, N-halogenated sulfonamides, N-halogenated imides, hypochlorites and mixtures thereof.

7. Process according to claim 6, wherein step (g) is carried out in the presence of at least one inorganic or organic acid and at least one equimolar amount of water, based on the compound of formula (VIII) or (VIIIa).

8. Process according to any one of claims 1 to 7, wherein step (h) is carried out in a mixture of an organic solvent and water in the presence of an azidation agent.

9. Process according to claim 8, wherein step (h) is carried out in the presence of a phase transfer catalyst.

10. Process according to any one of claims 1 to 9, wherein the metal catalyst used in step (j1) or step (12) is iron acetonyl acetate.

11. Process according to claim 10, wherein step (j1) or step (12) is carried out in N-methylpyrrolidone.

12. Process according to claim 10 or 11, wherein step (j1) or step (12) is carried out at a temperature of 50°C to 80°C.

13. Process according to any one of claims 10 to 12, wherein in step (j1) the compound of formula (XII) is firstly reacted with a metal, preferably an earth alkaline metal such as magnesium, and the obtained Grignard product is then reacted with the compound of formula (XI) in the presence of said metal catalyst.

14. Process according to any one of claims 10 to 12, wherein in step (j1) the compound of formula (XI) is firstly reacted with a metal, preferably an earth alkaline metal such as magnesium, and the obtained Grignard product is then reacted with the compound of formula (XII) in the presence of said metal catalyst.

15. Process according to any one of claims 10 to 12, wherein in step (12) the compound of formula (XII) is firstly reacted with a metal, preferably an earth alkaline metal such as magnesium, and the obtained Grignard product is then reacted with the compound of formula (XVIII) in the presence of said metal catalyst.

16. Process according to any one of claims 10 to 12, wherein in step (12) the compound of formula (XVIII) is firstly reacted with a metal, preferably an earth alkaline metal such as magnesium, and the obtained Grignard product is then reacted with the compound of formula (XII) in the presence of said metal catalyst.

17. Process according to any one of claims 1 to 12, 15 or 16, wherein in step (i2) the compound of formula (X) is reacted with a compound selected from the group consisting of tert.-butyldimethylsilylchloride, trimethylsilylchloride, triethylsilylchloride, triisopropylsilylchloride and tert.-butyldiphenylsilylchloride.

18. Process according to any one of claims 1 to 12 or 15 to 17, wherein step (j2) is carried out in the presence of a compound selected from the group consisting of 2-hydroxypyridine, N-hydroxycarboxamides, N-hydroxycarboximides such as N-hydroxysuccinimide, and mixtures thereof.

19. Process according to claim 18, wherein in step (j2) a tertiary amine such as trimethyl amine and/or triethyl amine is used as a solvent.

20. Process for preparing aliskiren of the following formula (I) or a pharmaceutically acceptable salt thereof 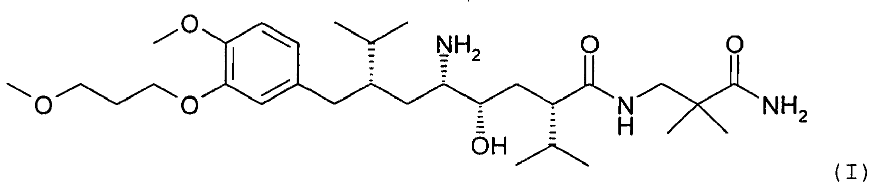 comprising
converting a compound of formula (II) 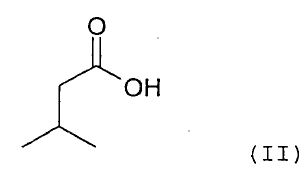 into a compound of formula (III) 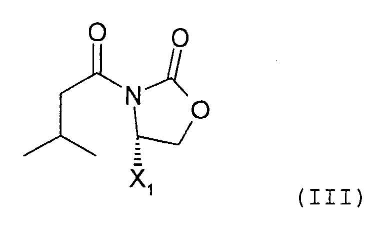 wherein X₁ is an alkylaryl group,
and converting the compound (III) into a compound of formula (I).

21. Process for preparing aliskiren of the following formula (I) or a pharmaceutically acceptable salt thereof 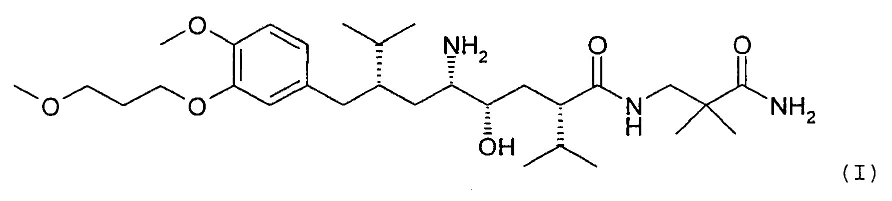 comprising
reacting a compound of formula (III) 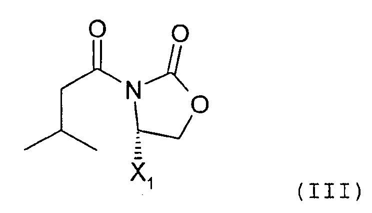 wherein X₁ is an alkylaryl group,
with 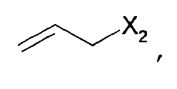 wherein X₂ is halogen, to form a compound of formula (IV) 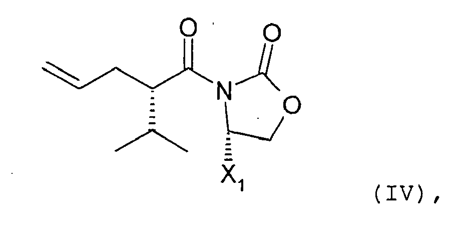 and converting the compound (IV) into a compound of formula (I).

22. Process for preparing aliskiren of the following formula (I) or a pharmaceutically acceptable salt thereof 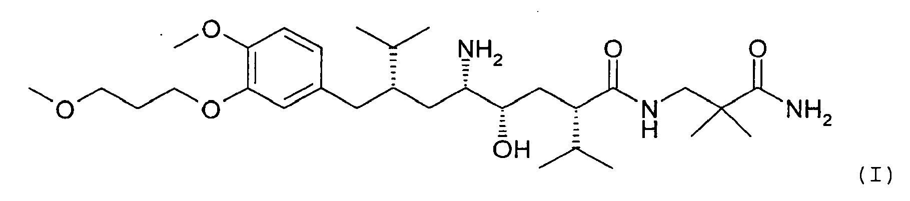 comprising
converting a compound of formula (IV) 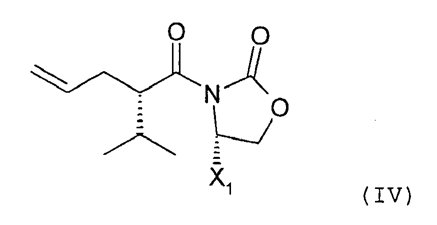 wherein X₁ is an alkylaryl group,
into a compound of formula (V) 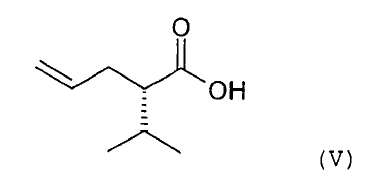 and converting the compound (V) into a compound of formula (I).

23. Process for preparing aliskiren of the following formula (I) or a pharmaceutically acceptable salt thereof 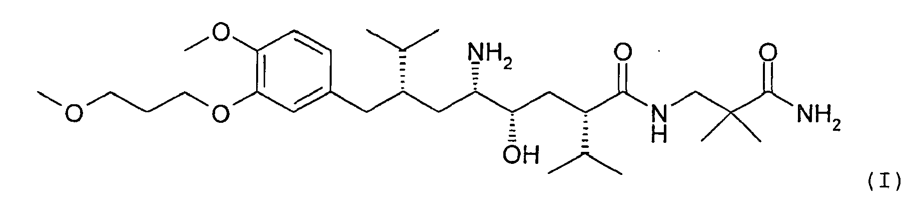 comprising
converting a compound of formula (V) 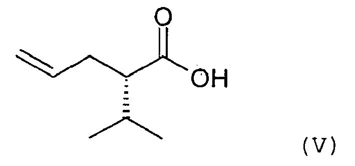 into a compound of formula (VI) 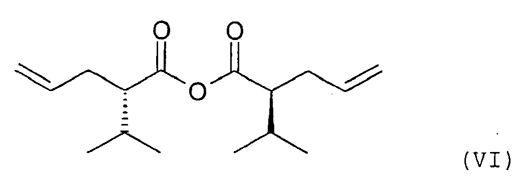 and converting the compound (VI) into a compound of formula (I).

24. Process for preparing aliskiren of the following formula (I) or a pharmaceutically acceptable salt thereof 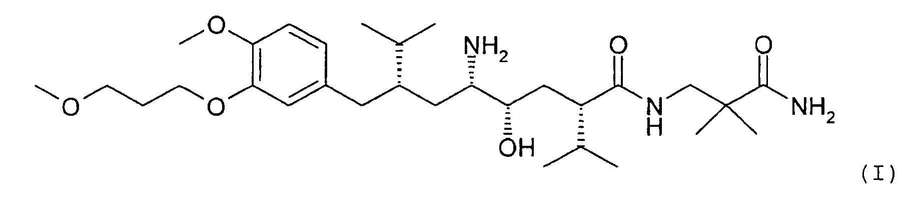 comprising
cyclizing a compound of formula (VI) 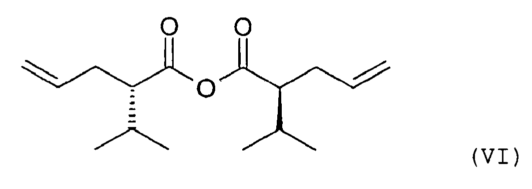 in the presence of a metal catalyst to form a compound of formula (VII) 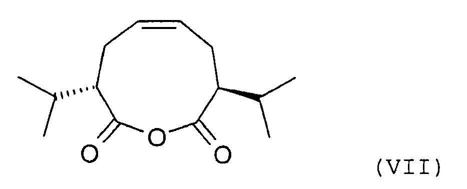 and converting the compound (VII) into a compound of formula (I).

25. Process for preparing aliskiren of the following formula (I) or a pharmaceutically acceptable salt thereof 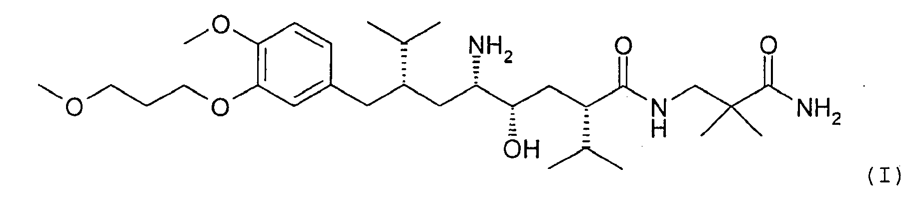 comprising
converting a compound of formula (VII) 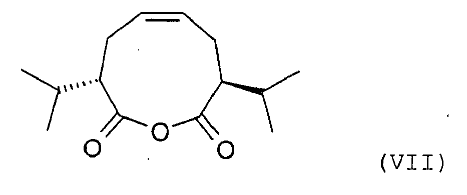 into a compound of formula (VIII) or (VIIIa) 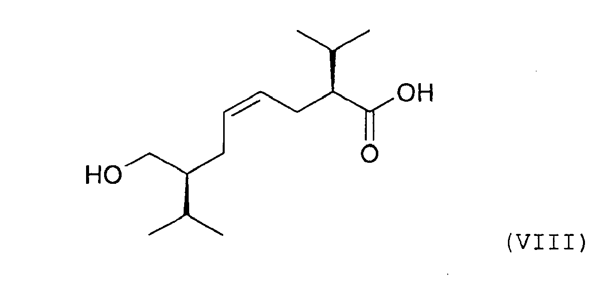 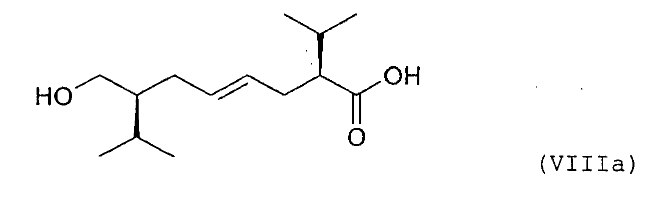 and converting the compound (VIII) or (VIIIa) into a compound of formula (I).

26. Process for preparing aliskiren of the following formula (I) or a pharmaceutically acceptable salt thereof 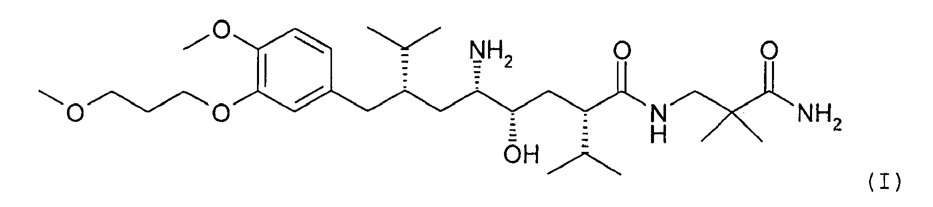 comprising
lactonizing a compound of formula (VIII) or (VIIIa) 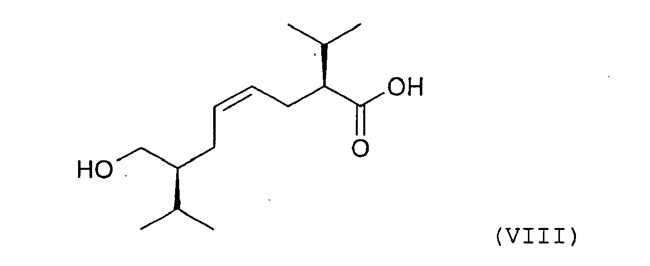 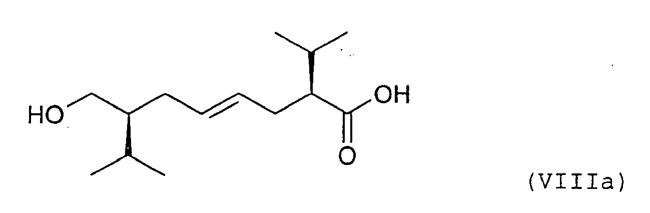 into a compound of formula (IX) 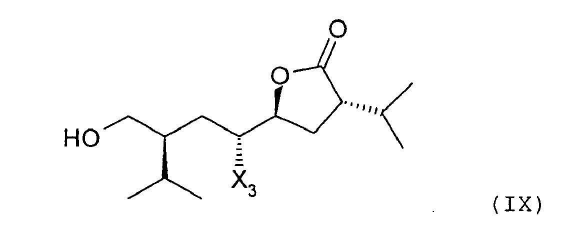 wherein X₃ is halogen, and converting the compound (IX) into a compound of formula (I).

27. Process for preparing aliskiren of the following formula (I) or a pharmaceutically acceptable salt thereof 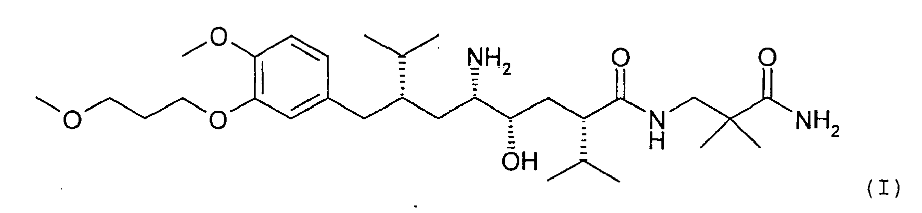 comprising
azidating a compound of formula (IX) 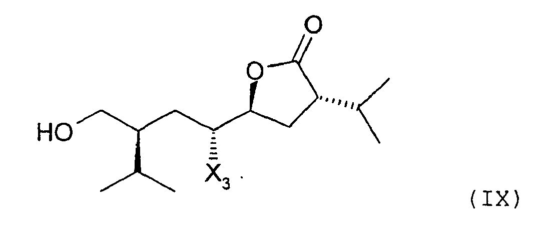 wherein X₃ is halogen,
to form a compound of formula (X) 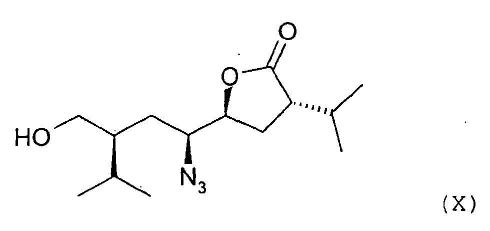 and converting the compound (X) into a compound of formula (I).

28. Process for preparing aliskiren of the following formula (I) or a pharmaceutically acceptable salt thereof 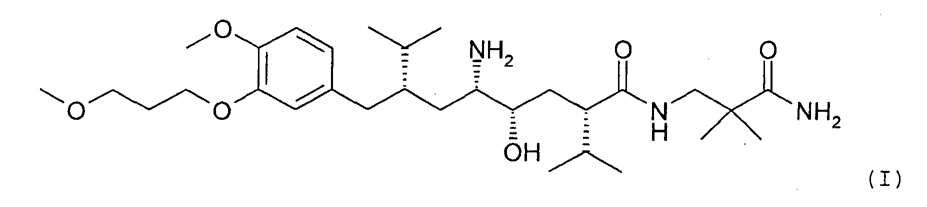 comprising
converting a compound of formula (X) 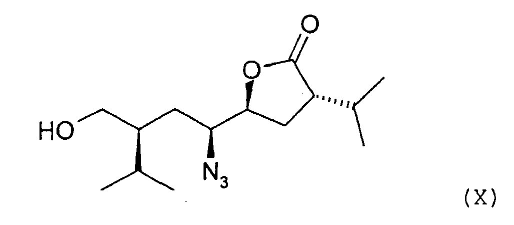 into a compound of formula (XI) 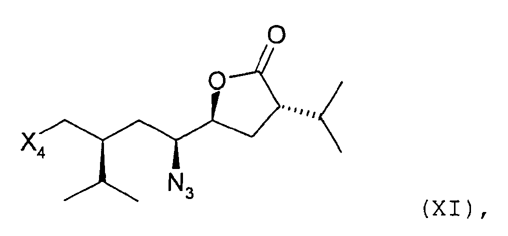 wherein X₄ is halogen,
and converting the compound (XI) into a compound of formula (I).

29. Process for preparing aliskiren of the following formula (I) or a pharmaceutically acceptable salt thereof 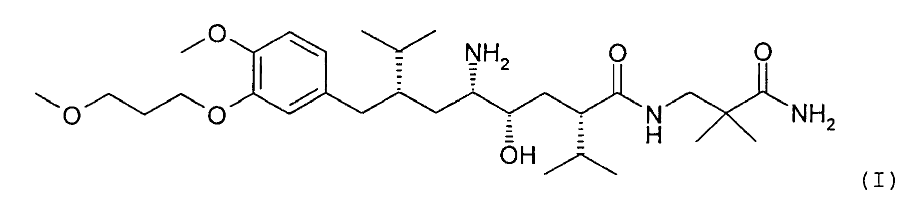 comprising
reacting a compound of formula (XI) 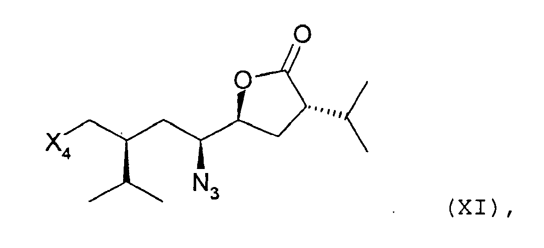 wherein X₄ is halogen,
with a compound of formula (XII) in the presence of a metal catalyst 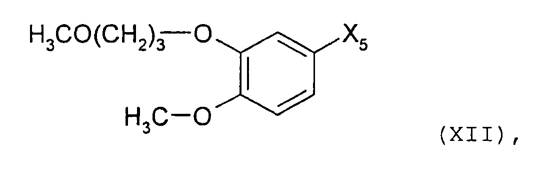 wherein X₅ is halogen,
to form a compound of formula (XIII) 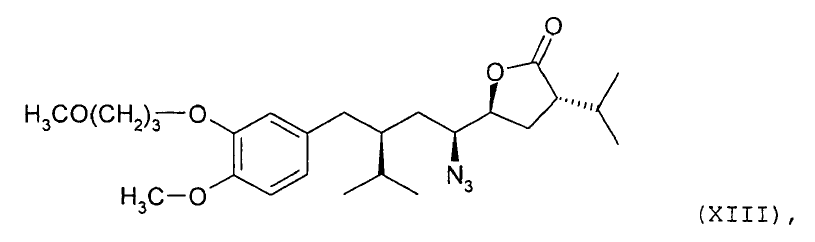 and converting the compound (XIII) into a compound of formula (I).

30. Process for preparing aliskiren of the following formula (I) or a pharmaceutically acceptable salt thereof 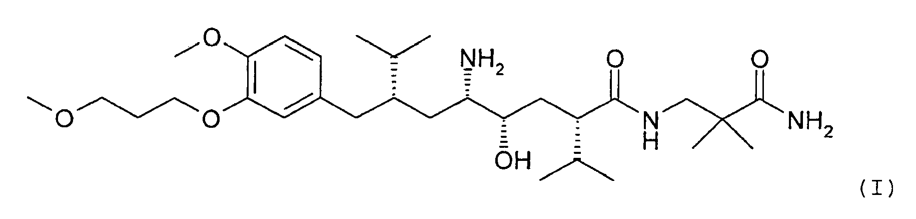 comprising
converting a compound of formula (X) 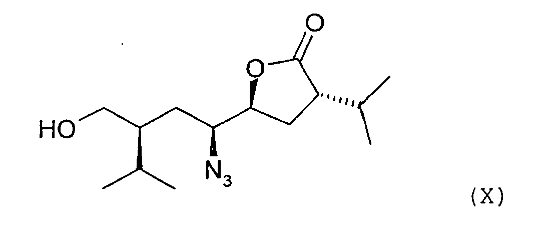 into a compound of formula (XVI) 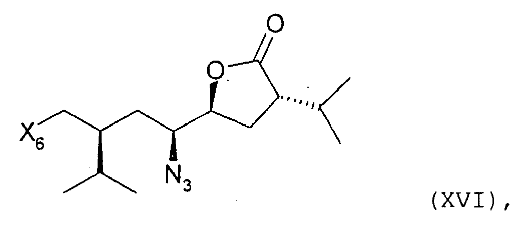 wherein X₆ is selected from the group consisting of trialkylsilyl, dialkylarylsilyl, alkyldiarylsilyl and triarylsilyl,
and converting the compound (XVI) into a compound of formula (I).

31. Process for preparing aliskiren of the following formula (I) or a pharmaceutically acceptable salt thereof 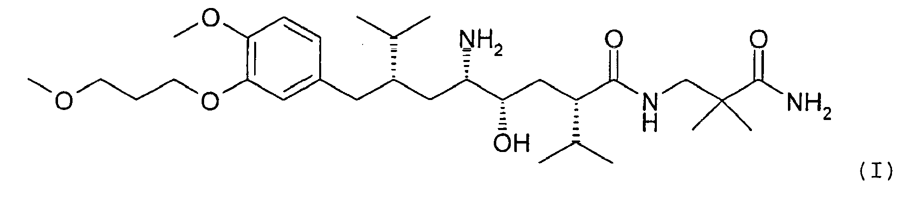 comprising
reacting a compound of formula (XVI) 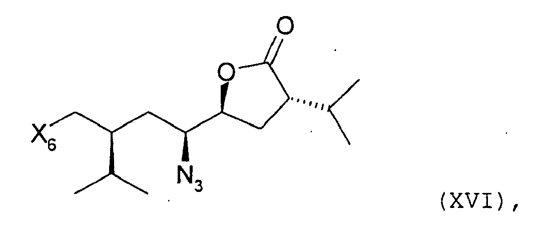 wherein X₆ is selected from the group consisting of trialkylsilyl, dialkylarylsilyl, alkyldiarylsilyl and triarylsilyl,
with an amine of formula (XIV) 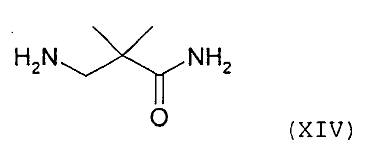 to form a compound of formula (XVII) 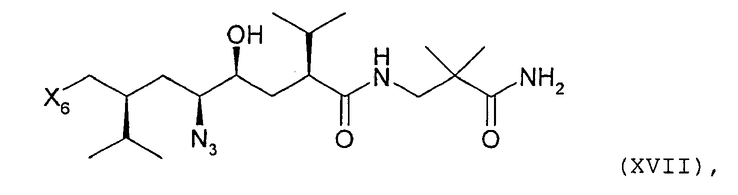 and converting the compound (XVII) into a compound of formula (I).

32. Process for preparing aliskiren of the following formula (I) or a pharmaceutically acceptable salt thereof 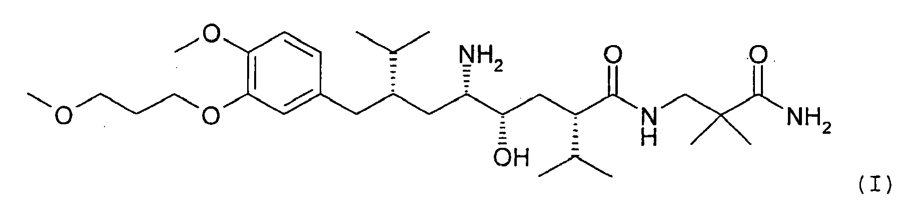 comprising
converting a compound of formula (XVII) 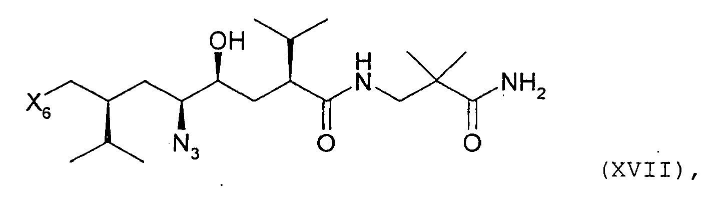 wherein X₆ is selected from the group consisting of trialkylsilyl, dialkylarylsilyl, alkyldiarylsilyl and triarylsilyl,
into a compound of formula (XVIII) 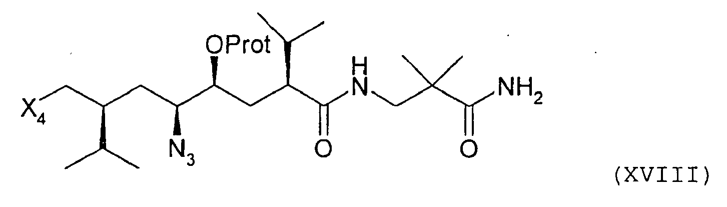 wherein X₄ is halogen and Prot is a hydroxyl protecting group,
and converting the compound (XVIII) into a compound of formula (I).

33. Process for preparing aliskiren of the following formula (I) or a pharmaceutically acceptable salt thereof 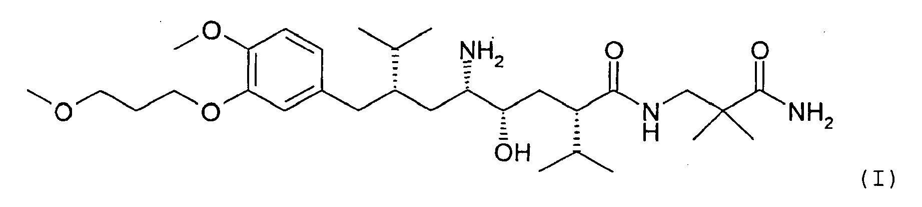 comprising
reacting a compound of formula (XVIII) 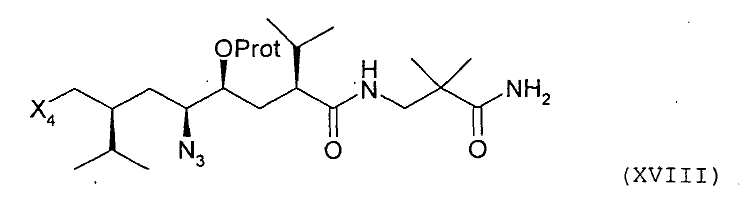 wherein X₄ is halogen and Prot is a hydroxyl protecting group,
with a compound of formula (XII) in the presence of a metal catalyst 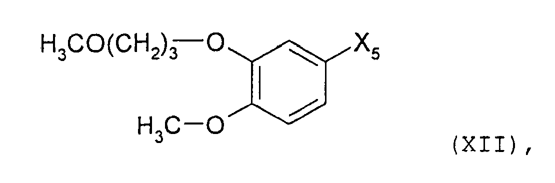 wherein X₅ is halogen,
to form a compound of formula (XIX) 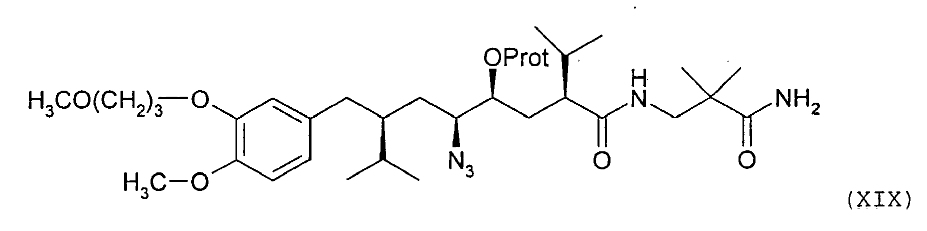 and converting the compound (XIX) into a compound of formula (I).

34. A compound of formula (VI)

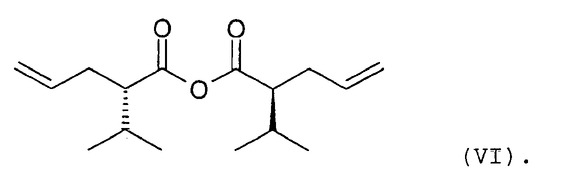

35. A compound of formula (VII)

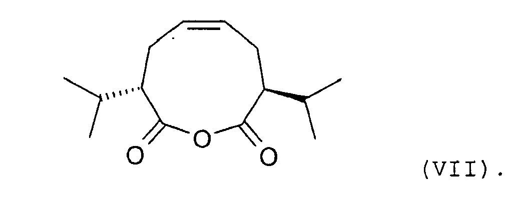

36. A compound of formula (VIII) or (VIIIa)

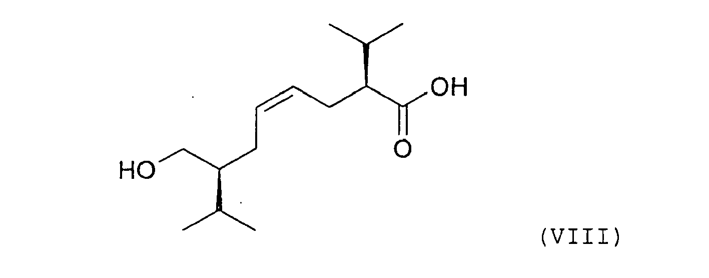

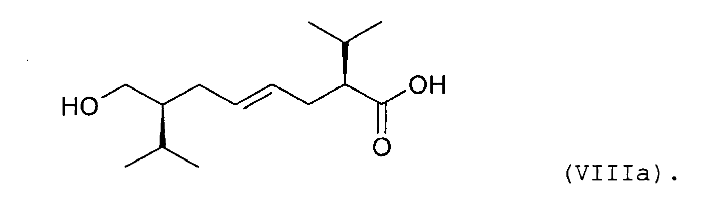

37. A compound of formula (IX) 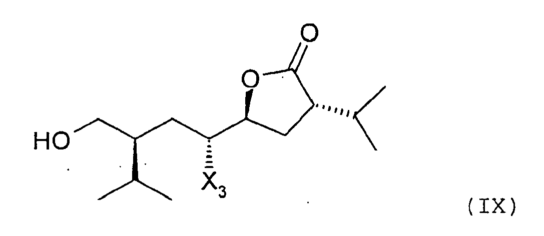 wherein X₃ is halogen.

38. A compound of formula (XI) 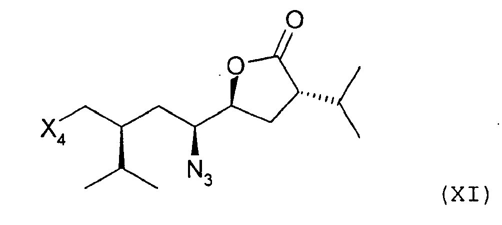 wherein X₄ is halogen.

39. A compound of formula (XVI) 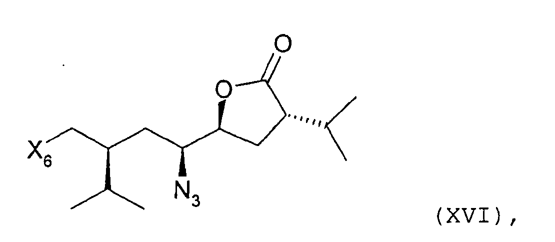 wherein X₆ is selected from the group consisting trialkylsilyl, dialkylarylsilyl, alkyldiarylsilyl and triarylsilyl.

40. A compound of formula (XVII) 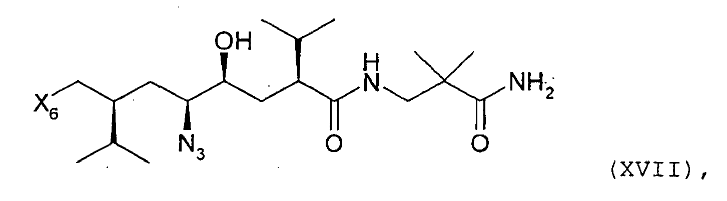 wherein X₆ is selected from the group consisting of trialkylsilyl, dialkylarylsilyl, alkyldiarylsilyl and triarylsilyl.

41. A compound of formula (XVIII) 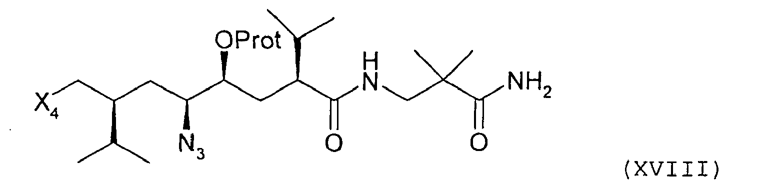 wherein X₄ is halogen and Prot is a hydroxyl protecting group.

42. Use of a compound according to any one of claims 34 to 41 for preparing aliskiren of the following formula (I) or a pharmaceutically acceptable salt thereof

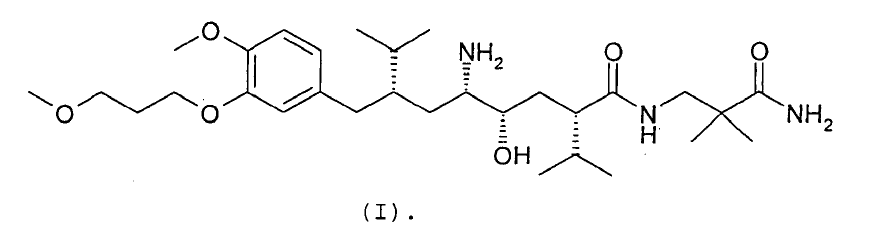

43. Process for preparing aliskiren of the following formula (I) or a pharmaceutically acceptable salt thereof 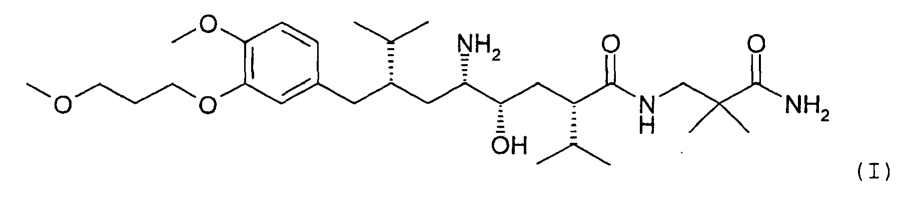 using at least one compound according to any one of claims 34 to 41.

44. Process for preparing a pharmaceutical composition containing aliskiren or a pharmaceutically acceptable salt thereof, comprising preparing aliskiren or a pharmaceutically acceptable salt thereof in accordance with a process of any one of claims 1 to 33 or 43 and converting the aliskiren or salt thereof into the pharmaceutical composition.

45. Process according to claim 44, wherein the conversion of aliskiren or a pharmaceutically acceptable salt thereof into the pharmaceutical composition is effected by a hotmelt and solvent-free granulation process.

## Patentansprüche

1. Verfahren zur Herstellung von Aliskiren der folgenden Formel (I) oder eines pharmazeutisch annehmbaren Salzes davon 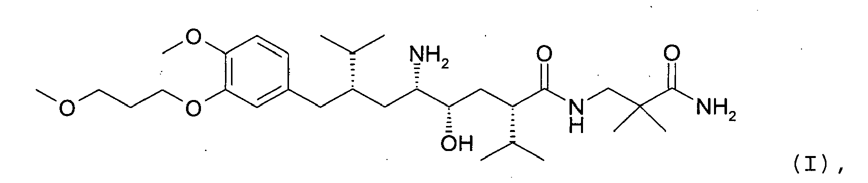 bei dem
(a) eine Verbindung der Formel (II) 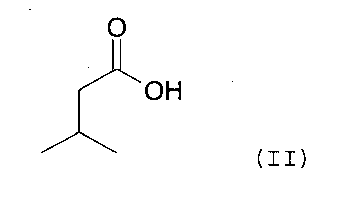 in eine Verbindung der Formel (III) 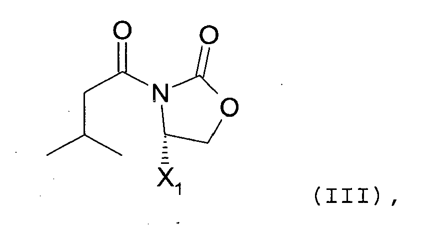 in der X₁ eine Alkylarylgruppe ist, umgewandelt wird,
(b) die Verbindung der Formel (III) mit 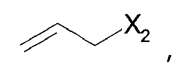 in der X₂ Halogen ist, umgesetzt wird, um eine Verbindung der Formel (IV) 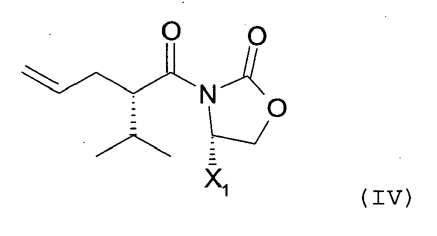 zu bilden,
(c) die Verbindung der Formel (IV) in eine Verbindung der Formel (V) 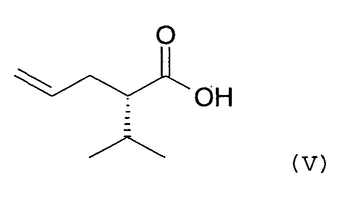 umgewandelt wird,
(d) die Verbindung der Formel (V) in eine Verbindung der Formel (VI) 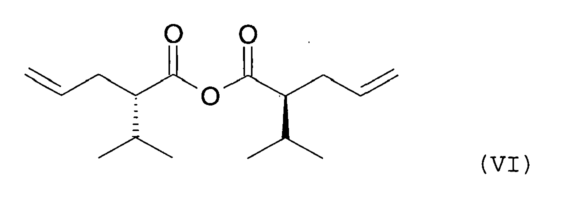 umgewandelt wird,
(e) die Verbindung der Formel (VI) in Gegenwart eines Metallkatalysators cyclisiert wird, um eine Verbindung der Formel (VII) 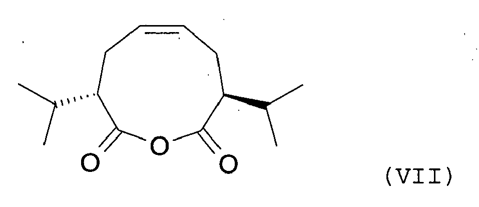 zu bilden,
(f) die Verbindung der Formel (VII) in eine Verbindung der Formel (VIII) oder (VIIIa) 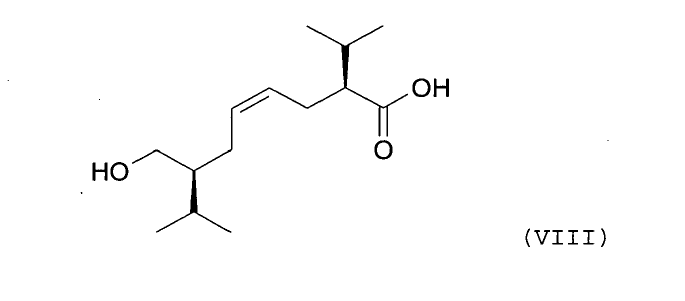 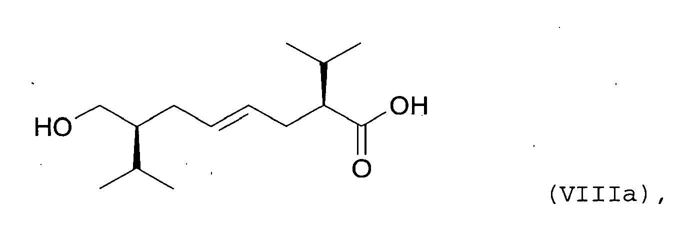 umgewandelt wird,
(g) die Verbindung der Formel (VIII) oder (VIIIa) in eine Verbindung der Formel (IX) 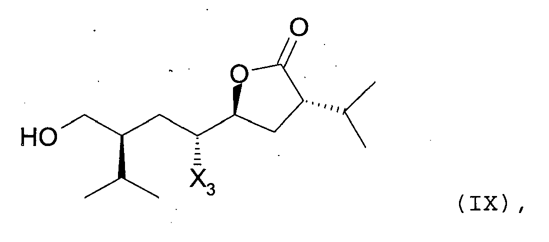 in der X₃ Halogen ist, laktonisiert wird,
(h) die Verbindung der Formel (IX) azidiert wird, um eine Verbindung der Formel (X) 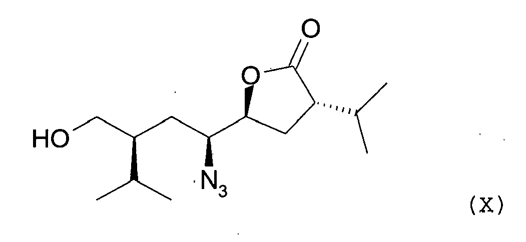 zu bilden,
und entweder
(i1) die Verbindung der Formel (X) in eine Verbindung der Formel (XI) 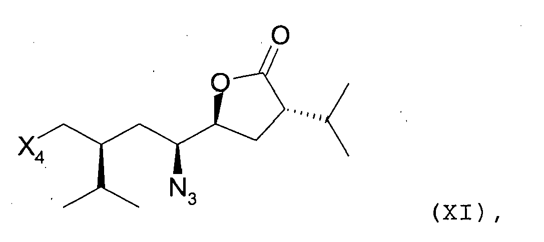 in der X₄ Halogen ist, umgewandelt wird,
(j1) die Verbindung der Formel (XI) mit einer Verbindung der Formel (XII) 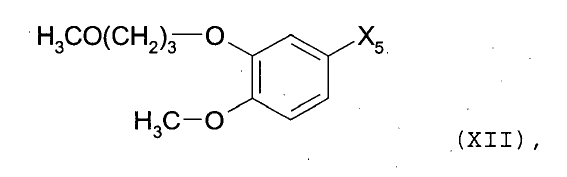 in der X₅ Halogen ist, in Gegenwart eines Metallkatalysators umgesetzt wird, um eine Verbindung der Formel (XIII) 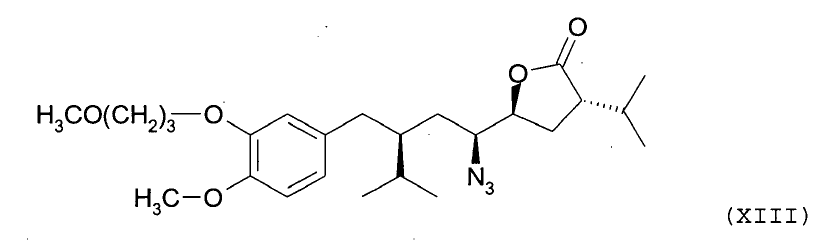 zu bilden,
(k1) die Verbindung der Formel (XIII) mit einem Amin der Formel (XIV) 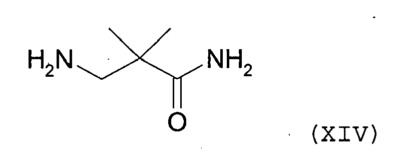 umgesetzt wird, um eine Verbindung der. Formel (XV) 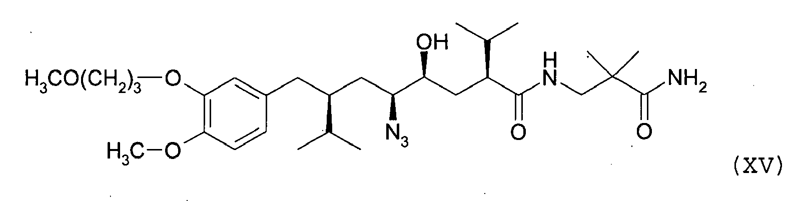 zu bilden,
und
(l1) die Verbindung der Formel (XV) in die Verbindung der Formel (I) oder ein pharmazeutisch annehmbares Salz davon umgewandelt wird,
oder
(i2) die Verbindung der Formel (X) in eine Verbindung der Formel (XVI) 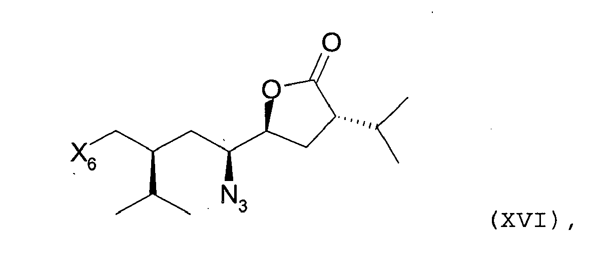 in der X₆ ausgewählt ist aus der Gruppe bestehend aus Trialkylsilyl, Dialkylarylsilyl, Alkyldiarylsilyl und Triarylsilyl, umgewandelt wird,
(j2) die Verbindung der Formel (XVI) mit einem Amin der Formel (XIV) 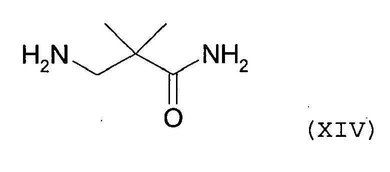 umgesetzt wird, um eine Verbindung der Formel (XVII) 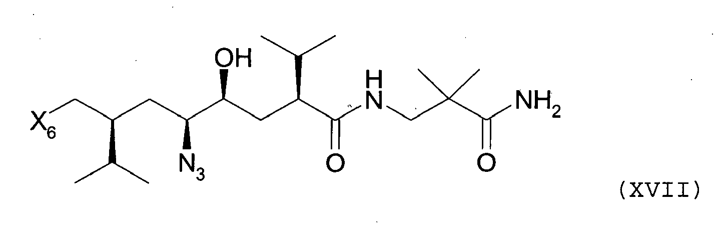 zu bilden,
(k2) die Verbindung der Formel (XVII) in eine Verbindung der Formel (XVIII) 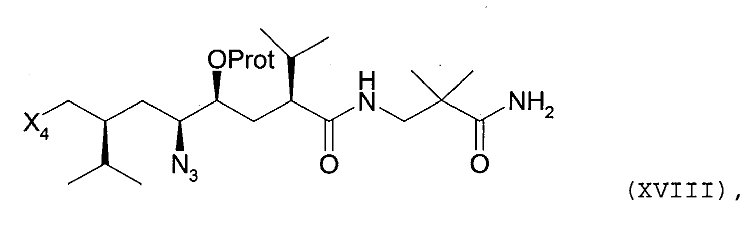 in der X₄ Halogen und Prot eine Hydroxyl-Schutzgruppe ist, umgewandelt wird,
(l2) die Verbindung der Formel (XVIII) mit einer Verbindung der Formel (XII) 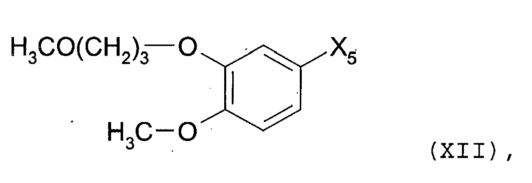 in der X₅ Halogen ist, in Gegenwart eines Metallkatalysators umgesetzt wird, um eine Verbindung der Formel (XIX) 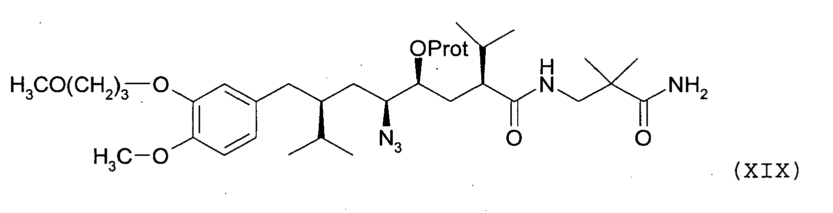 zu bilden
und
(m2) die Verbindung der Formel (XIX) in die Verbindung der Formel (I) oder ein pharmazeutisch annehmbares Salz davon umgewandelt wird.

2. Verfahren nach Anspruch 1, bei dem die Verbindung der Formel (II) in Schritt (a) in Isovaleriansäurehalogenid umgewandelt wird und das Isovaleriansäurehalogenid mit einem (S)-4-X₁-Oxazilidon-2-on-Anion umgesetzt wird.

3. Verfahren nach Anspruch 1 oder 2, bei dem die Verbindung der Formel (V) in Schritt (d) in Gegenwart von Essigsäureanhydrid und/oder P₂O₅ auf eine Temperatur zwischen Raumtemperatur und der Siedetemperatur der Reaktionsmischung erhitzt wird.

4. Verfahren nach einem der Ansprüche 1 bis 3, bei dem der Metallkatalysator in Schritt (e) ein Grubbs-Katalysator, wie beispielsweise Cl₂(P(cymene)₃)₂Ru=CHPh, ist und die Reaktionsmischung auf eine Temperatur zwischen Raumtemperatur und der Siedetemperatur der Reaktionsmischung erhitzt wird.

5. Verfahren nach einem der Ansprüche 1 bis 4, bei dem die Verbindung der Formel (VII) in Schritt (f) durch ein Reduktionsmittel, bevorzugt Zn/HOAc, H₂/Pt, RuCl₂(PhP)₃, NaBH₄ oder LiAlH₄, reduziert wird und dann zu einer Verbindung der Formel (VIII) oder (VIIIa) hydrolisiert wird.

6. Verfahren nach einem der Ansprüche 1 bis 5, bei dem die Verbindung der Formel (VIII) oder (VIII_{A}) in Schritt (g) in Gegenwart eines Halogenierungsmittels, das ausgewählt ist aus der Gruppe bestehend aus Br₂, I₂, N-halogenierten Carboxamiden, N-halogenierten Dicarboxamiden, N-halogenierten Phthalimiden, N-halogenierten Succinimiden, N-halogenierten Sulfonamiden, N-halogenierten Imiden, Hypochloriten und Mischungen davon, laktonisiert wird.

7. Verfahren nach Anspruch 6, bei dem Schritt (g) in Gegenwart mindestens einer anorganischen oder organischen Säure und mindestens einer äquimolaren Menge Wasser, bezogen auf die Verbindung der Formel (VIII) oder (VIIIa), ausgeführt wird.

8. Verfahren nach einem der Ansprüche 1 bis 7, bei dem Schritt (h) in einem Gemisch eines organischen Lösemittels und Wasser in Gegenwart eines Azidierungsmittels ausgeführt wird.

9. Verfahren nach Anspruch 8, bei dem Schritt (h) in Gegenwart eines Phasentransfer-Katalysators ausgeführt wird.

10. Verfahren nach einem der Ansprüche 1 bis 9, bei dem der in Schritt (j1) oder Schritt (j2) verwendete Metallkatalysator Eisenacetonylacetat ist.

11. Verfahren nach Anspruch 10, bei dem Schritt (j1) oder Schritt (l2) in N-Methylpyrrolidon ausgeführt wird.

12. Verfahren nach Anspruch 10 oder 11, bei dem Schritt (j1) oder Schritt (l2) bei einer Temperatur von 50°C bis 80°C ausgeführt wird.

13. Verfahren nach einem der Ansprüche 10 bis 12, bei dem die Verbindung der Formel (XII) in Schritt (j1) zunächst mit einem Metall, bevorzugt ein Erdalkalimetall wie Magnesium, umgesetzt wird und das erhaltene Grignard-Produkt dann mit der Verbindung der Formel (XI) in Gegenwart des Metallkatalysators umgesetzt wird.

14. Verfahren nach einem der Ansprüche 10 bis 12, bei dem die Verbindung der Formel (XI) in Schritt (j1) zunächst mit einem Metall, bevorzugt ein Erdalkalimetall wie Magnesium, umgesetzt wird und das erhaltene Grignard-Produkt dann mit der Verbindung der Formel (XII) in Gegenwart des Metallkatalysators umgesetzt wird.

15. Verfahren nach einem der Ansprüche 10 bis 12, bei dem die Verbindung der Formel (XII) in Schritt (12) zunächst mit einem Metall, bevorzugt ein Erdalkalimetall wie Magnesium, umgesetzt wird und das erhaltene Grignard-Produkt dann mit der Verbindung der Formel (XVIII) in Gegenwart des Metallkatalysators umgesetzt wird.

16. Verfahren nach einem der Ansprüche 10 bis 12, bei dem die Verbindung der Formel (XVIII) in Schritt (l2) zunächst mit einem Metall, bevorzugt ein Erdalkalimetall wie Magnesium, umgesetzt wird und das erhaltene Grignard-Produkt dann mit der Verbindung der Formel (XII) in Gegenwart des Metallkatalysators umgesetzt wird.

17. Verfahren nach einem der Ansprüche 1 bis 12, 15 oder 16, bei dem die Verbindung der Formel (X) in Schritt (i2) mit einer Verbindung, die ausgewählt ist aus der Gruppe bestehend aus tert-Butyldimethylsilylchlorid, Trimethylsilylchlorid, Triethylsilylchlorid, Triisopropylsilylchlorid und tert-Butyldiphenylsilylchlorid, umgesetzt wird.

18. Verfahren nach einem der Ansprüche 1 bis 12 oder 15 bis 17, bei dem Schritt (j2) in Gegenwart einer Verbindung, die ausgewählt ist aus der Gruppe bestehend aus 2-Hydroxypyridin, N-Hydroxycarboxamiden, N-Hydroxycarboximiden wie N-Hydroxysuccinimid und Mischungen davon, ausgeführt wird.

19. Verfahren nach Anspruch 18, bei dem ein tertiäres Amin wie Trimethylamin und/oder Triethylamin in Schritt (j2) als Lösemittel verwendet wird.

20. Verfahren zur Herstellung von Alkiskiren der folgenden Formel (I) oder eines pharmazeutisch annehmbaren Salzes davon 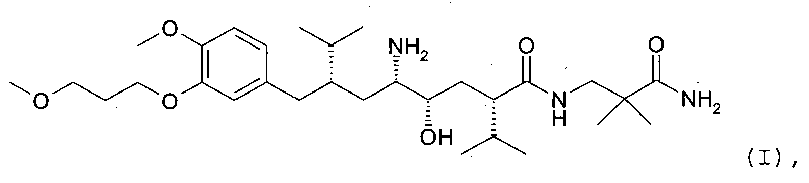 bei dem
eine Verbindung der Formel (II) 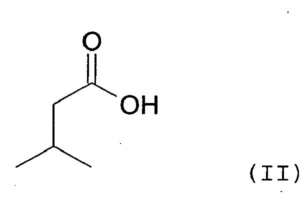 in eine Verbindung der Formel (III) 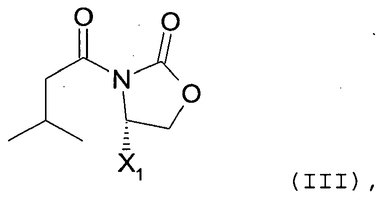 in der X₁ eine Alkylarylgruppe ist, umgewandelt wird
und
die Verbindung (III) in eine Verbindung der Formel (I) umgewandelt wird.

21. Verfahren zur Herstellung von Aliskiren der folgenden Formel (I) oder eines pharmazeutisch annehmbaren Salzes davon 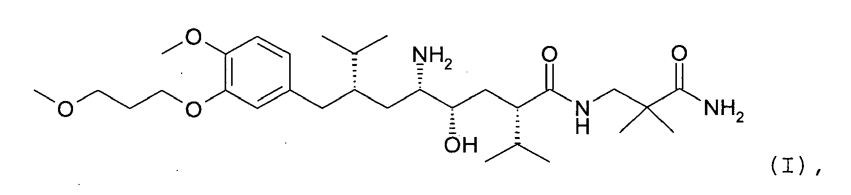 bei dem
eine Verbindung der Formel (III) 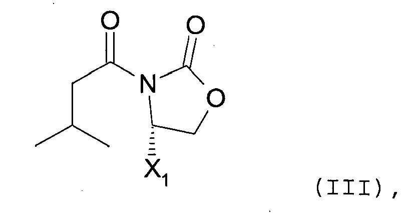 in der X₁ eine Alkylarylgruppe ist, mit 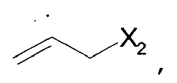 in der X₂ Halogen ist, umgesetzt wird, um eine Verbindung der Formel (IV) 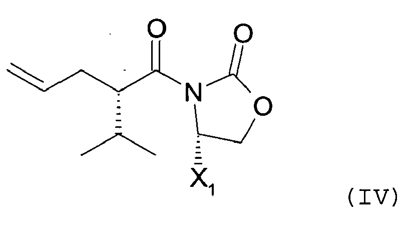 zu bilden und die Verbindung (IV) in eine Verbindung der Formel (I) umgewandelt wird.

22. Verfahren zur Herstellung von Aliskiren der folgenden Formel (I) oder eines pharmazeutisch annehmbaren Salzes davon 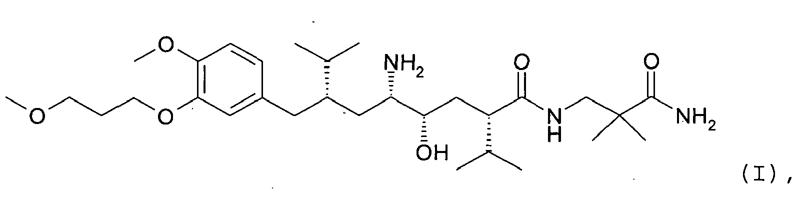 bei dem
eine Verbindung der Formel (IV) 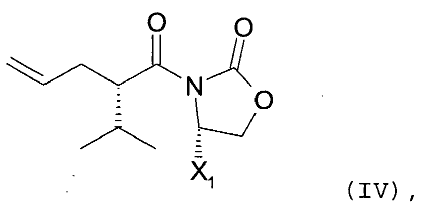 in der X₁ eine Alkylarylgruppe ist, in eine Verbindung der Formel (V) 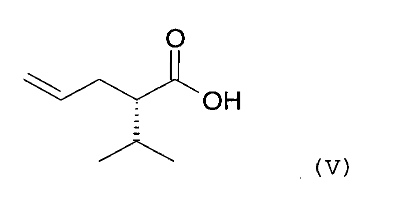 umgewandelt wird und die Verbindung (V) in eine Verbindung der Formel (I) umgewandelt wird.

23. Verfahren zur Herstellung von Aliskiren der folgenden Formel (I) oder eines pharmazeutisch annehmbaren Salzes davon 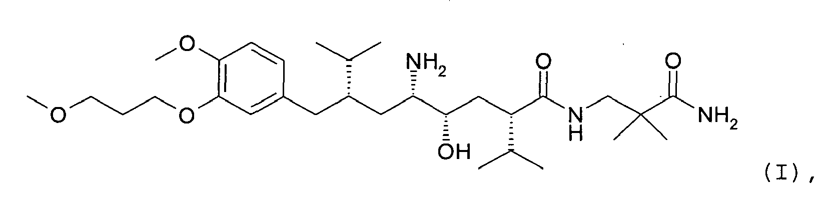 bei dem
eine Verbindung der Formel (V) 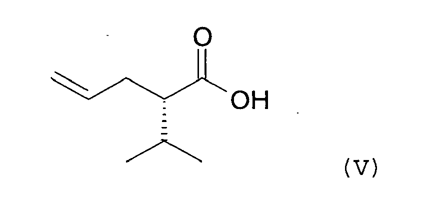 in eine Verbindung der Formel (VI) 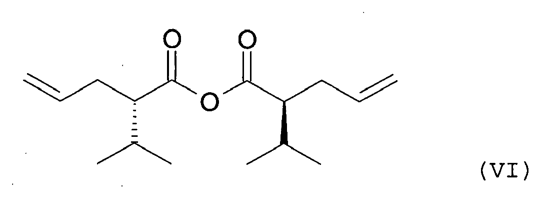 umgewandelt wird und die Verbindung (VI) in eine Verbindung der Formel (I) umgewandelt wird.

24. Verfahren zur Herstellung von Aliskiren der folgenden Formel (I) oder eines pharmazeutisch annehmbaren Salzes davon 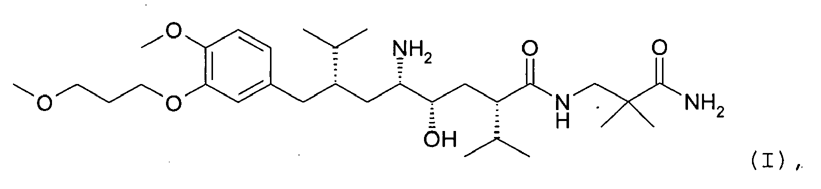 bei dem
eine Verbindung der Formel (VI) 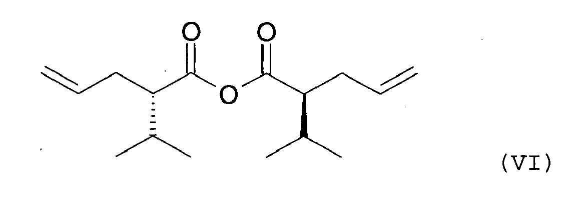 in Gegenwart eines Metallkatalysators cyclisiert wird, um eine Verbindung der Formel (VII) 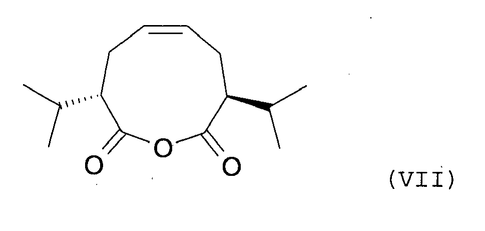 zu bilden und die Verbindung (VII) in eine Verbindung der Formel (I) umgewandelt wird.

25. Verfahren zur Herstellung von Aliskiren der folgenden Formel (I) oder eines pharmazeutisch annehmbaren Salzes davon 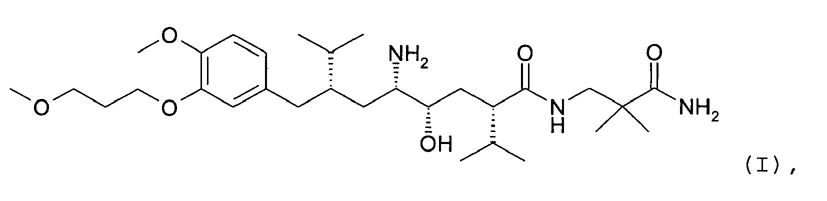 bei dem
eine Verbindung der Formel (VII) 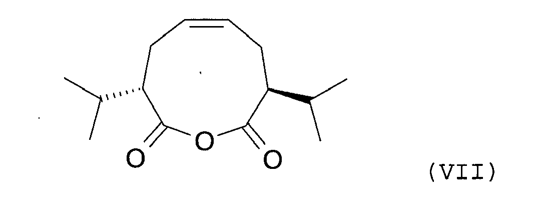 in eine Verbindung der Formel (VIII) oder (VIIIa) 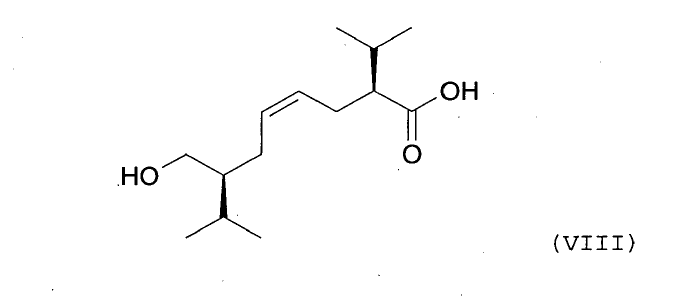 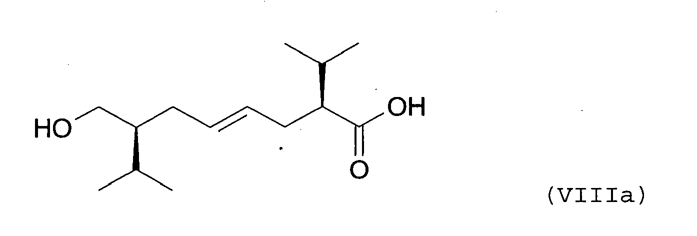 umgewandelt wird und die Verbindung (VIII) oder (VIIIa) in eine Verbindung der Formel (I) umgewandelt wird.

26. Verfahren zur Herstellung von Aliskiren der folgenden Formel (I) oder eines pharmazeutisch annehmbaren Salzes davon 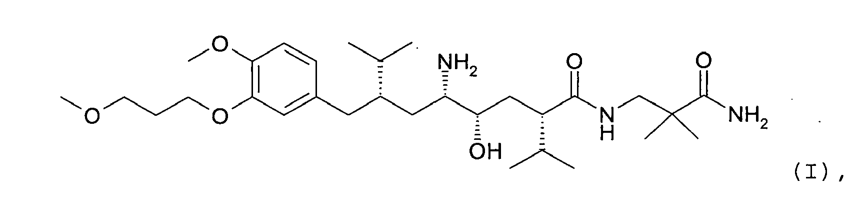 bei dem
eine Verbindung der Formel (VIII) oder (VIIIa) 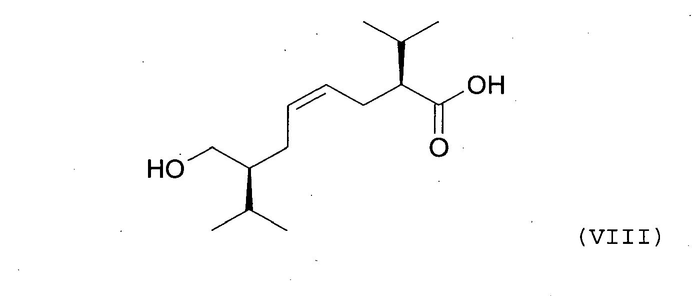 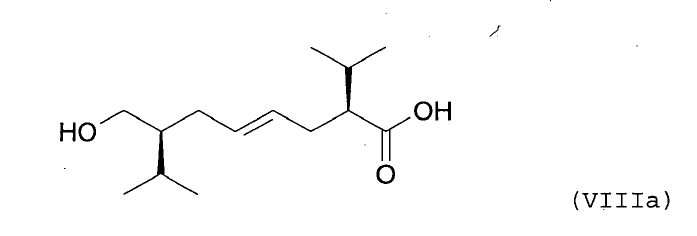 in eine Verbindung der Formel (IX) 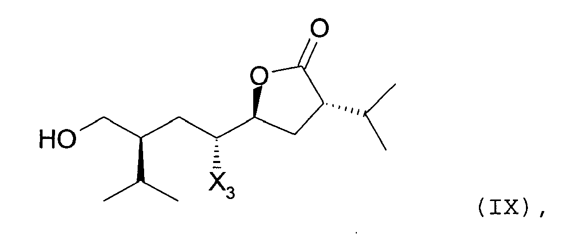 in der X₃ Halogen ist, laktonisiert wird und die Verbindung (IX) in eine Verbindung der Formel (I) umgewandelt wird.

27. Verfahren zur Herstellung von Aliskiren der folgenden Formel (I) oder eines pharmazeutisch annehmbaren Salzes davon 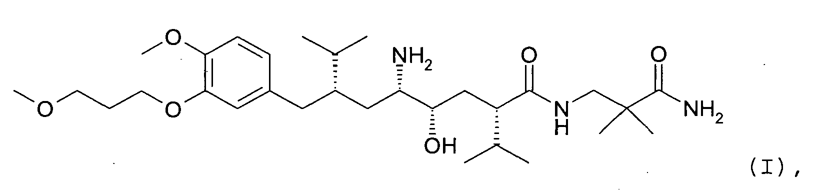 bei dem
eine Verbindung der Formel (IX) 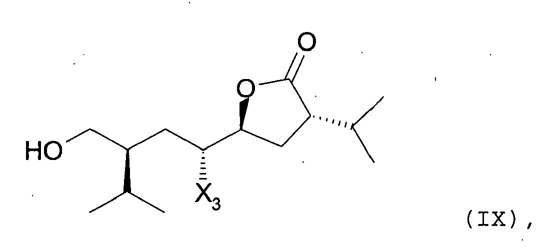 in der X₃ halogen ist, azidiert wird, um eine Verbindung der Formel (X) 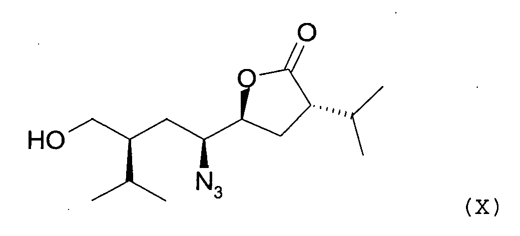 zu bilden und die Verbindung (X) in eine Verbindung der Formel (I) umgewandelt wird.

28. Verfahren zur Herstellung von Aliskiren der folgenden Formel (I) oder eines pharmazeutisch annehmbaren Salzes davon 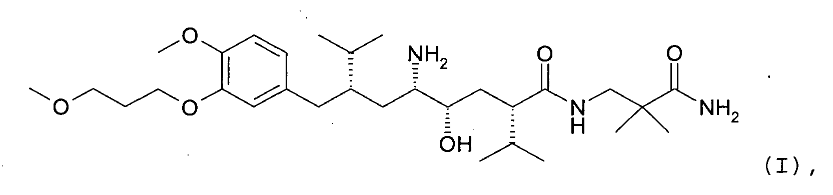 bei dem eine Verbindung der Formel (X) 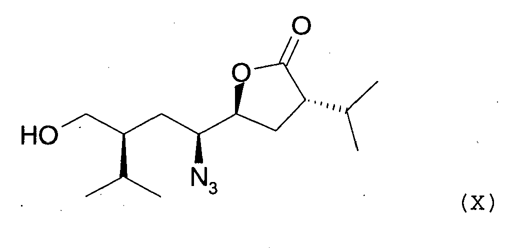 in eine Verbindung der Formel (XI) 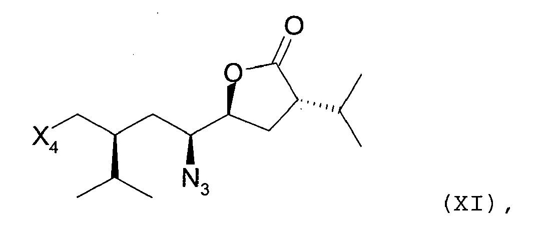 in der X₄ Halogen ist, umgewandelt wird und die Verbindung (XI) in eine Verbindung der Formel (I) umgewandelt wird.

29. Verfahren zur Herstellung von Aliskiren der folgenden Formel (I) oder eines pharmazeutisch annehmbaren Salzes davon 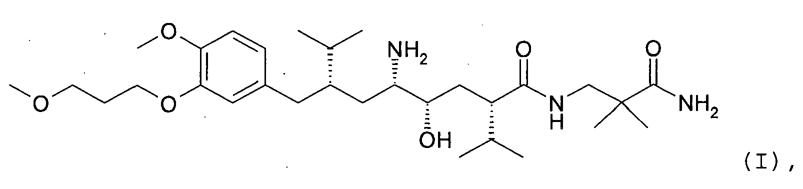 bei dem
eine Verbindung der Formel (XI) 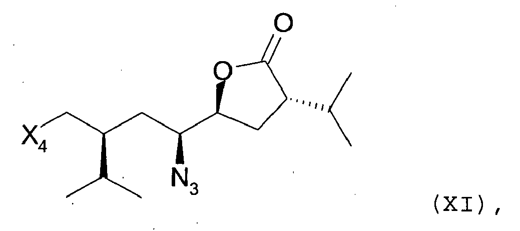 in der X₄ Halogen ist, in Gegenwart eines Metallkatalysators mit einer Verbindung der Formel (XII) 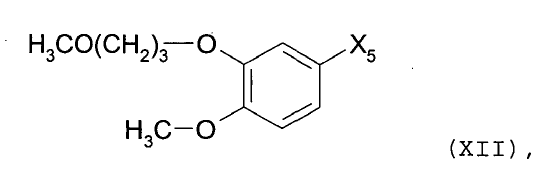 in der X₅ Halogen ist, umgesetzt wird, um eine Verbindung der Formel (XIII) 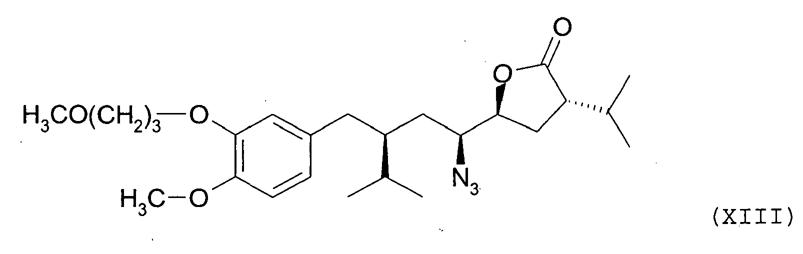 zu bilden und die Verbindung (XIII) in eine Verbindung der Formel (I) umgesetzt wird.

30. Verfahren zur Herstellung von Aliskiren der folgenden Formel (I) oder eines pharmazeutisch annehmbaren Salzes davon 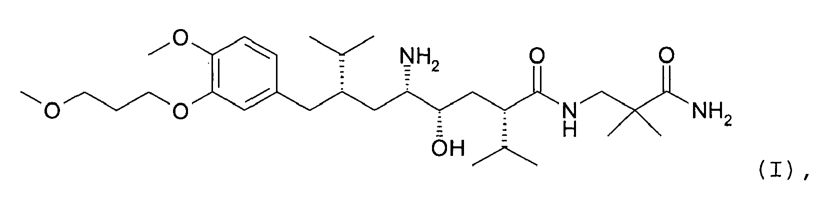 bei dem
eine Verbindung der Formel (X) 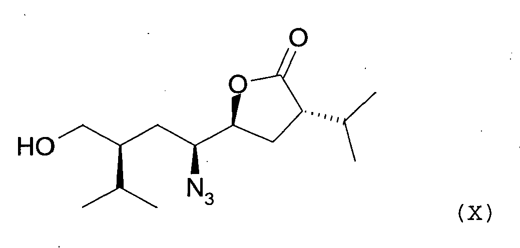 in eine Verbindung der Formel (XVI) 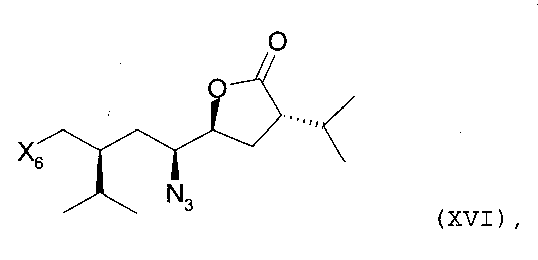 in der X₆ ausgewählt ist aus der Gruppe bestehend aus Trialkylsilyl, Dialkylarylsilyl, Alkyldiarylsilyl und Triarylsilyl, umgewandelt wird und die Verbindung (XVI) in eine Verbindung der Formel (I) umgewandelt wird.

31. Verfahren zur Herstellung von Aliskiren der folgenden Formel (I) oder eines pharmazeutisch annehmbaren Salzes davon 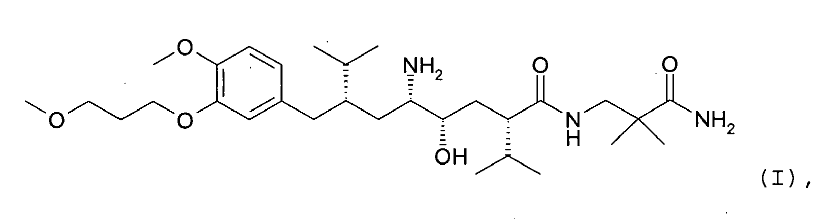 bei dem
eine Verbindung der Formel (XVI) 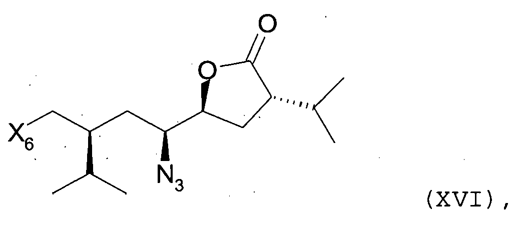 in der X₆ ausgewählt ist aus der Gruppe bestehend aus Trialkylsilyl, Dialkylarylsilyl, Alkyldiarylsilyl und Triarylsilyl, mit einem Amin der Formel (XIV) 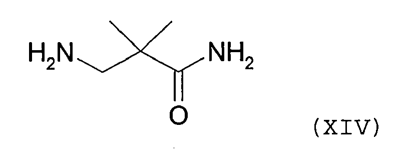 umgesetzt wird, um eine Verbindung der Formel (XVII) 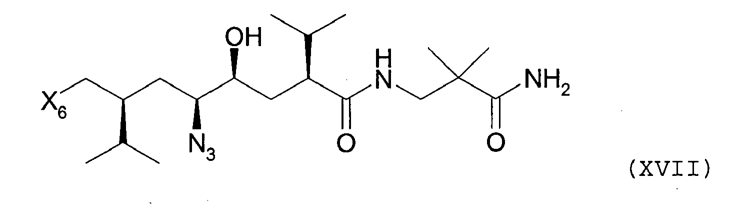 zu erhalten und die Verbindung (XVII) in eine Verbindung der Formel (I) umgesetzt wird.

32. Verfahren zur Herstellung von Aliskiren der folgenden Formel (I) oder eines pharmazeutisch annehmbaren Salzes davon 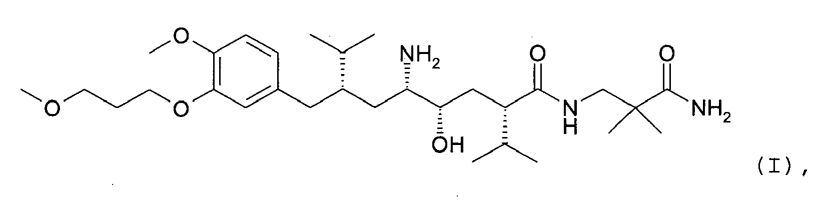 bei dem
eine Verbindung der Formel (XVII) 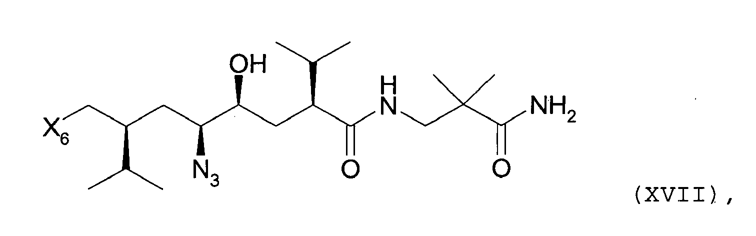 in der X₆ ausgewählt ist aus der Gruppe bestehend aus Trialkylsilyl, Dialkylarylsilyl, Alkyldiarylsilyl und Triarylsilyl, in eine Verbindung der Formel (XVIII) 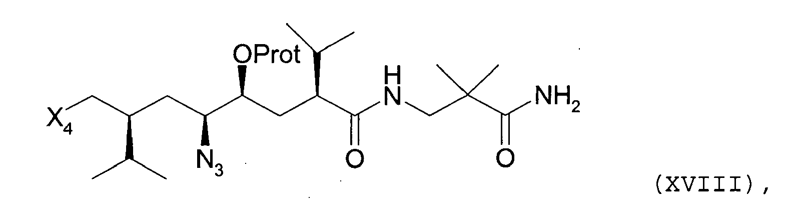 in der X₄ Halogen und Prot eine Hydroxyl-Schutzgruppe ist, umgewandelt wird und die Verbindung (XVIII) in eine Verbindung der Formel (I) umgewandelt wird.

33. Verfahren zur Herstellung von Aliskiren der folgenden Formel (I) oder eines pharmazeutisch annehmbaren Salzes davon 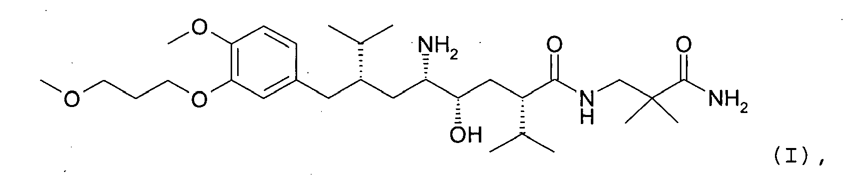 bei dem
eine Verbindung der Formel (XVIII) 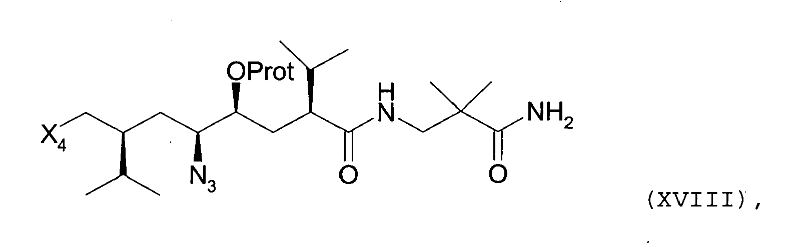 in der X₄ Halogen und Prot eine Hydroxyl-Schutzgruppe ist, in Gegenwart eines Metallkatalysators mit einer Verbindung der Formel (XII) 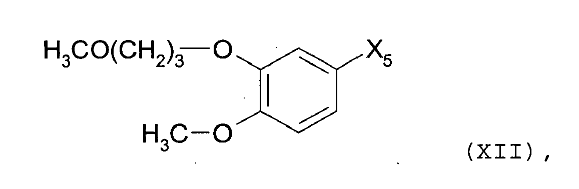 in der X₅ Halogen ist, umgesetzt wird, um eine Verbindung der Formel (XIX) 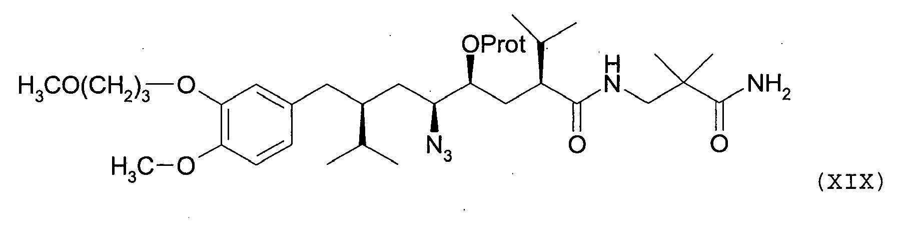 zu bilden und die Verbindung (XIX) in eine Verbindung der Formel (I) umgewandelt wird.

34. Verbindung der Formel (VI)

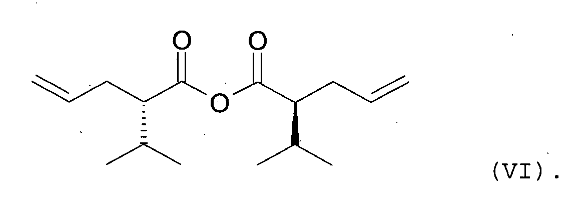

35. Verbindung der Formel (VII)

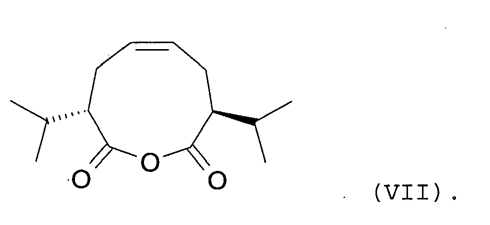

36. Verbindung der Formel (VIII) oder (VIIIa)

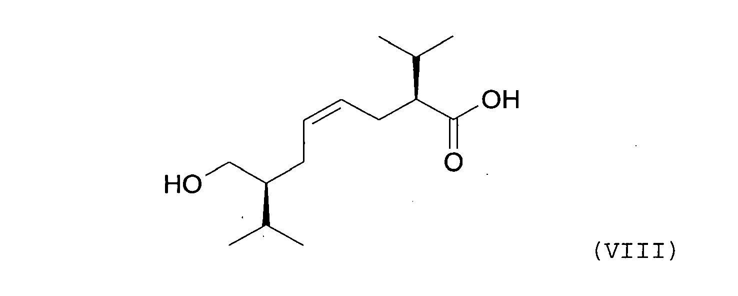

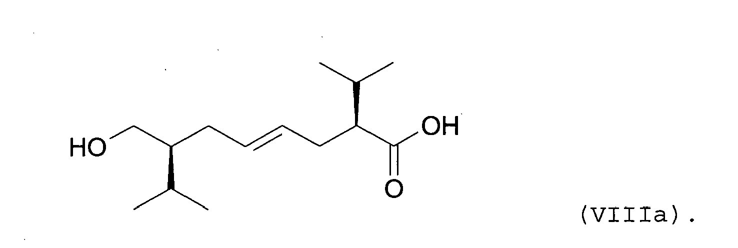

37. Verbindung der Formel (IX) 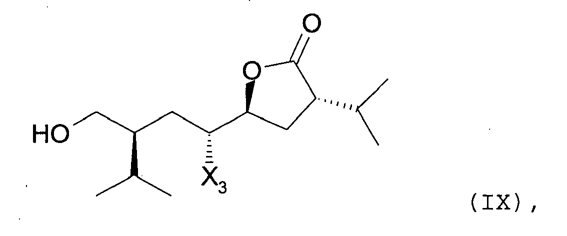 in der X3 Halogen ist.

38. Verbindung der Formel (XI) 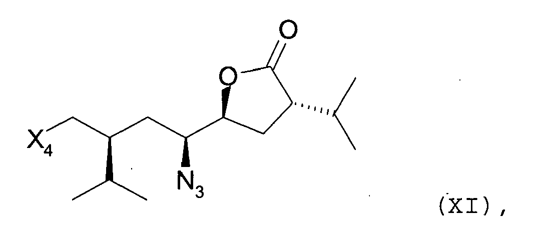 in der X₄ Halogen ist.

39. Verbindung der Formel (XVI) 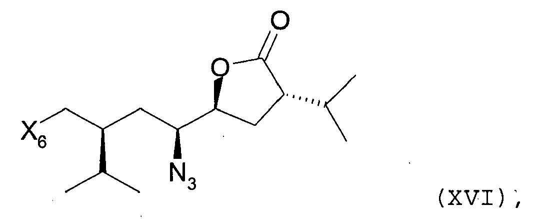 in der X₆ ausgewählt ist aus der Gruppe bestehend aus Trialkylsilyl, Dialkylarylsilyl, Alkyldiarylsilyl und Triarylsilyl.

40. Verbindung der Formel (XVII) 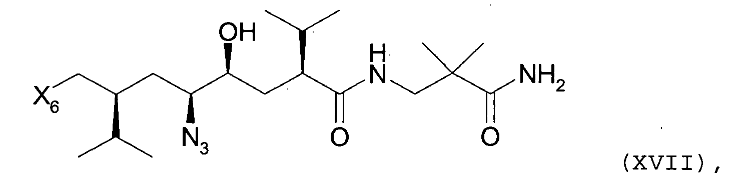 in der X₆ ausgewählt ist aus der Gruppe bestehend aus Trialkylsilyl, Dialkylarylsilyl, Alkyldiarylsilyl und Triarylsilyl.

41. Verbindung der Formel (XVIII) 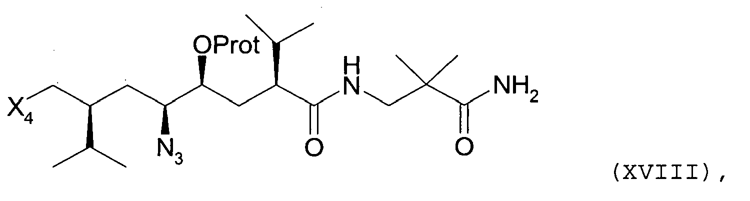 in der X₄ Halogen und Prot eine Hydroxyl-Schutzgruppe ist.

42. Verwendung einer Verbindung gemäß einem der Ansprüche 34 bis 41 zur Herstellung von Aliskiren der folgenden Formel (I) oder eines pharmazeutisch annehmbaren Salzes davon

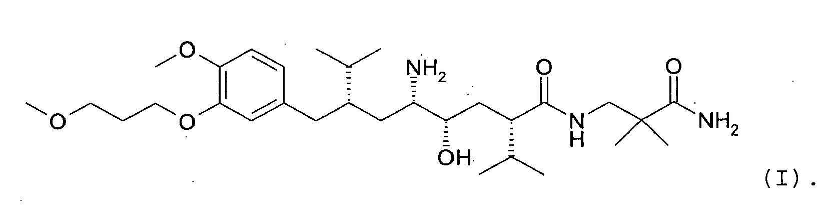

43. Verfahren zur Herstellung von Aliskiren der folgenden Formel (I) oder eines pharmazeutisch annehmbaren Salzes davon 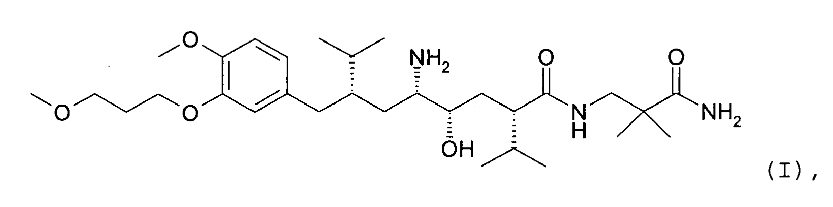 bei dem mindestens eine Verbindung gemäß einem der Ansprüche 34 bis 41 verwendet wird.

44. Verfahren zur Herstellung einer pharmazeutischen Zusammensetzung, die Aliskiren oder ein pharmazeutisch annehmbares Salz davon enthält, bei dem Aliskiren oder ein pharmazeutisch annehmbares Salz davon gemäß einem Verfahren einer der Ansprüche 1 bis 33 oder 43 hergestellt wird und Aliskiren oder ein Salz davon in eine pharmazeutische Zusammensetzung umgewandelt wird.

45. Verfahren nach Anspruch 44, bei dem die Umwandlung von Aliskiren oder eines pharmazeutisch annehmbaren Salzes davon durch ein lösemittelfreies Schmelzgranulierungsverfahren erfolgt.

## Revendications

1. Procédé de préparation de l'aliskirène, de formule (I) suivante, ou d'un sel pharmacologiquement admissible de ce composé : 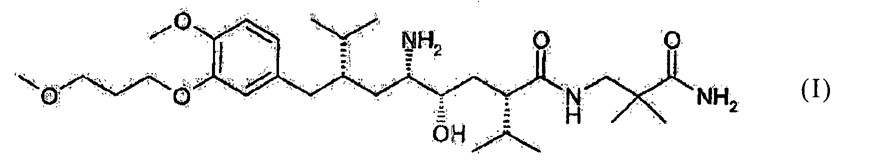 lequel procédé comporte les étapes suivantes :
a) convertir un composé de formule (II) 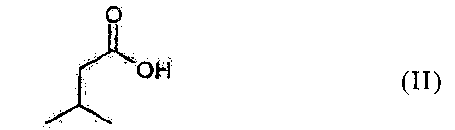 en un composé de formule (III) 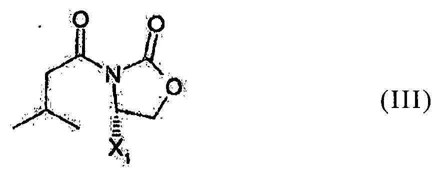 dans laquelle X₁ représente un groupe alkyl-aryle ;
b) faire réagir ce composé de formule (III) avec un composé de formule 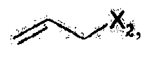 dans laquelle X₂ représente un atome d'halogène, de manière à obtenir un composé de formule (IV) : 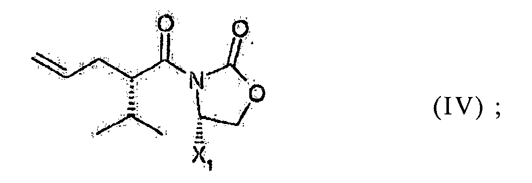
c) convertir ce composé de formule (IV) en un composé de formule (V) : 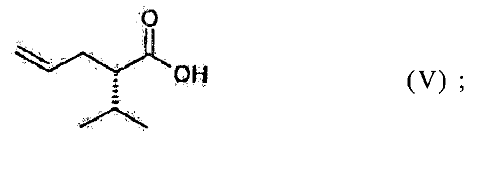
d) convertir ce composé de formule (V) en un composé de formule (VI) : 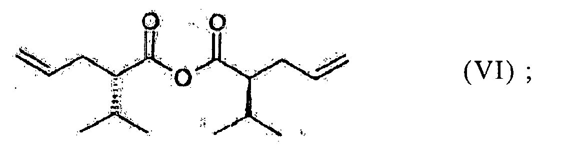
e) opérer la cyclisation de ce composé de formule (VI), en présence d'un catalyseur à base de métal, de manière à obtenir un composé de formule (VII) : 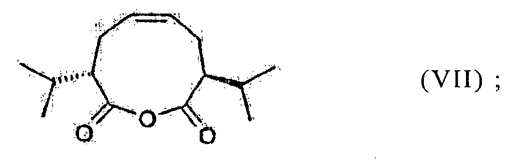
f) convertir ce composé de formule (VII) en un composé de formule (VIII) ou (VIIIa) : 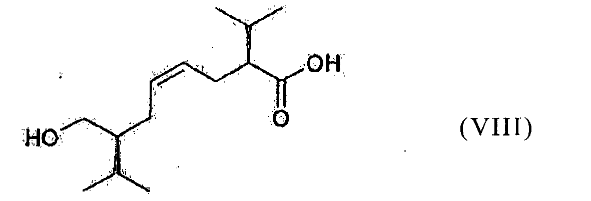 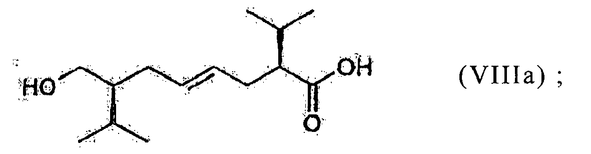
g) convertir en lactone ce composé de formule (VIII) ou (VIIIa), de manière à obtenir un composé de formule (IX) : 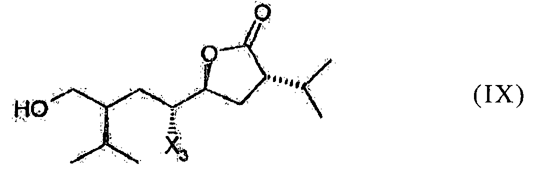 dans laquelle X₃ représente un atome d'halogène ;
h) convertir en azoture ce composé de formule (IX), de manière à obtenir un composé de formule (X) : 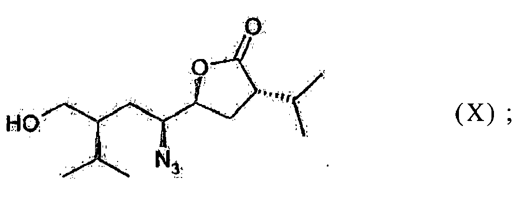 et soit :
i1) convertir ce composé de formule (X) en un composé de formule (XI) : 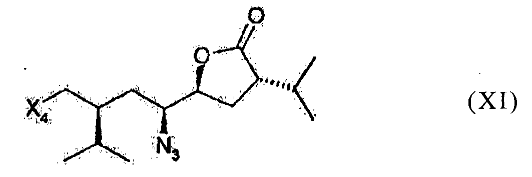 dans laquelle X₄ représente un atome d'halogène ;
j1) faire réagir ce composé de formule (XI), en présence d'un catalyseur à base de métal, avec un composé de formule (XII) : 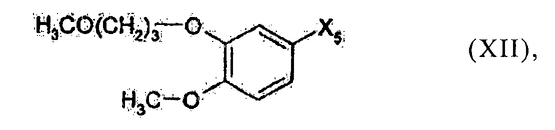 dans laquelle X₅ représente un atome d'halogène, de manière à obtenir un composé de formule (XIII) : 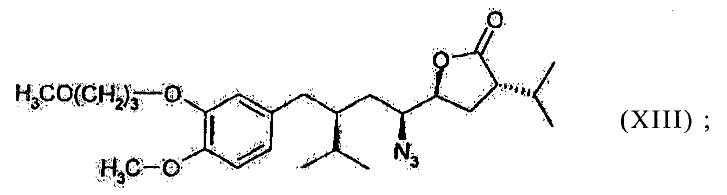
k1) faire réagir ce composé de formule (XIII) avec une amine de formule (XIV) : 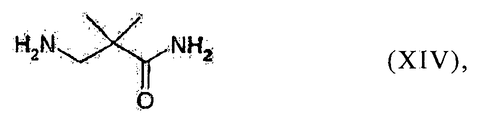 de manière à obtenir un composé de formule (XV) : 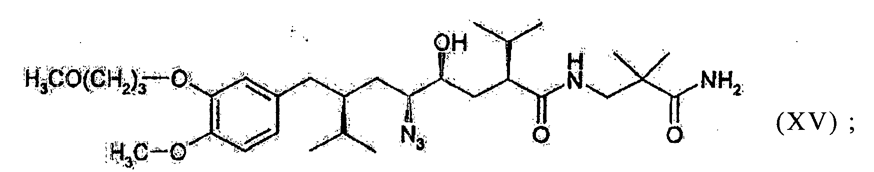
l1) et convertir ce composé de formule (XV) en le composé de formule (I) ou en un sel pharmacologiquement admissible de celui-ci ;
soit :
i2) convertir le composé de formule (X) en un composé de formule (XVI) : 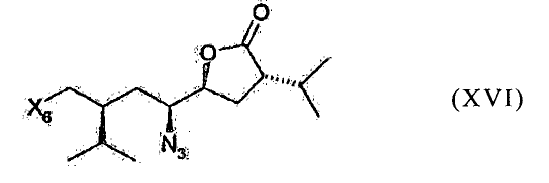 dans laquelle X₆ représente une entité choisie dans l'ensemble constitué par les groupes trialkyl-silyle, dialkyl-aryl-silyle, alkyl-diaryl-silyle et triaryl-silyle ;
j2) faire réagir ce composé de formule (XVI) avec une amine de formule (XIV) : 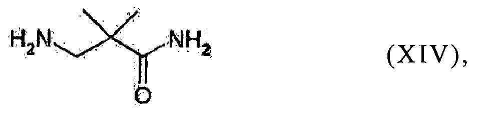 de manière à obtenir un composé de formule (XVII) : 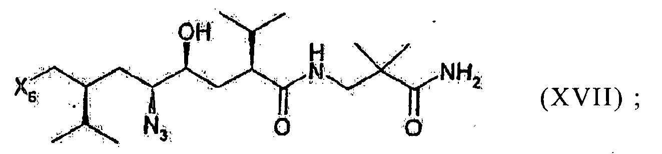
k2) convertir ce composé de formule (XVII) en un composé de formule (XVIII) : 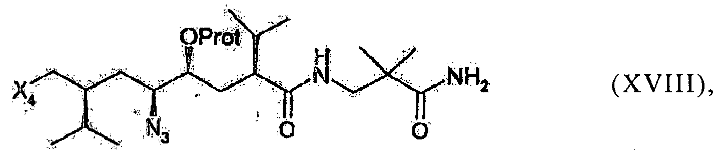 dans laquelle X₄ représente un atome d'halogène et Prot représente un groupe hydroxy-protecteur ;
l2) faire réagir ce composé de formule (XVIII), en présence d'un catalyseur à base de métal, avec un composé de formule (XII) : 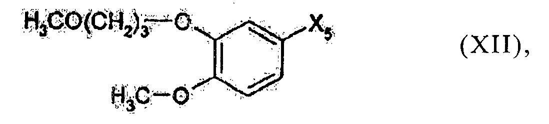 dans laquelle X₅ représente un atome d'halogène, de manière à obtenir un composé de formule (XIX) : 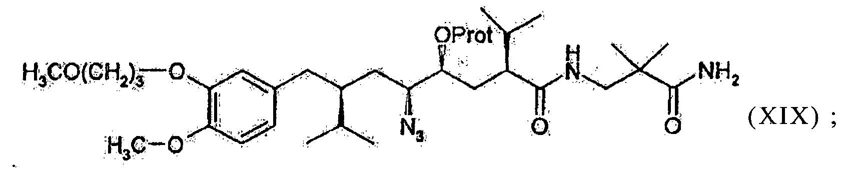
m2) et convertir ce composé de formule (XIX) en le composé de formule (I) ou en un sel pharmacologiquement admissible de celui-ci.

2. Procédé conforme à la revendication 1, dans lequel, dans l'étape (a), on convertit le composé de formule (II) en un halogénure de l'acide isovalérique, et l'on fait réagir cet halogénure d'acide isovalérique avec un anion (S)-4-X₁-oxazolidone-2-one.

3. Procédé conforme à la revendication 1 ou 2, dans lequel, dans l'étape (d), on chauffe le composé de formule (V), en présence d'anhydride acétique et/ou de pentoxyde de phosphore, à une température située dans l'intervalle allant de la température ambiante à la température d'ébullition du mélange réactionnel.

4. Procédé conforme à l'une des revendications 1 à 3, dans lequel, dans l'étape (e), le catalyseur à base de métal est un catalyseur de Grubbs, tel celui de formule Cl₂[P(cymène)₃]₂Ru=CHPh, et l'on chauffe le mélange réactionnel à une température située dans l'intervalle allant de la température ambiante à la température d'ébullition du mélange réactionnel.

5. Procédé conforme à l'une des revendications 1 à 4, dans lequel, dans l'étape (f), on réduit le composé de formule (VII) à l'aide d'un agent réducteur, de préférence Zn/HOAc, H₂/Pt, RuCl₂(PhP)₃, NaBH₄ ou LiAlH₄, puis on effectue une hydrolyse pour obtenir un composé de formule (VIII) ou (VIIIa).

6. Procédé conforme à l'une des revendications 1 à 5, dans lequel, dans l'étape (g), on convertit en lactone le composé de formule (VIII) ou (VIIIa), en présence d'un agent d'halogénation choisi dans l'ensemble formé par le brome Br₂, l'iode I₂, les carboxamides N-halogénés, les dicarboxamides N-halogénés, les phtalimides N-halogénés, les succinimides N-halogénés, les sulfonamides N-halogénés, les imides N-halogénés, les hypochlorites, et les mélanges de tels corps.

7. Procédé conforme à la revendication 6, dans lequel on réalise l'étape (g) en présence d'au moins un acide organique ou inorganique, et d'au moins une quantité équimolaire d'eau par rapport au composé de formule (VIII) ou (VIIIa).

8. Procédé conforme à l'une des revendications 1 à 7, dans lequel on réalise l'étape (h) dans un mélange d'eau et d'un solvant organique, en présence d'un agent de conversion en azoture.

9. Procédé conforme à la revendication 8, dans lequel on réalise l'étape (h) en présence d'un catalyseur par transfert de phase.

10. Procédé conforme à l'une des revendications 1 à 9, dans lequel le catalyseur à base de métal utilisé dans l'étape (j1) ou dans l'étape (12) est de l'acétonyl-acétate de fer.

11. Procédé conforme à la revendication 10, dans lequel on réalise l'étape (j1) ou l'étape (12) dans de la N-méthyl-pyrrolidone.

12. Procédé conforme à la revendication 10 ou 11, dans lequel on réalise l'étape (j1) ou l'étape (12) à une température de 50 à 80 °C.

13. Procédé conforme à l'une des revendications 10 à 12, dans lequel, dans l'étape (j1), on fait d'abord réagir le composé de formule (XII) avec un métal, de préférence avec un métal alcalino-terreux tel que du magnésium, et l'on fait ensuite réagir le produit de Grignard ainsi obtenu avec le composé de formule (XI), en présence dudit catalyseur à base de métal.

14. Procédé conforme à l'une des revendications 10 à 12, dans lequel, dans l'étape (j1), on fait d'abord réagir le composé de formule (XI) avec un métal, de préférence avec un métal alcalino-terreux tel que du magnésium, et l'on fait ensuite réagir le produit de Grignard ainsi obtenu avec le composé de formule (XII), en présence dudit catalyseur à base de métal.

15. Procédé conforme à l'une des revendications 10 à 12, dans lequel, dans l'étape (12), on fait d'abord réagir le composé de formule (XII) avec un métal, de préférence avec un métal alcalino-terreux tel que du magnésium, et l'on fait ensuite réagir le produit de Grignard ainsi obtenu avec le composé de formule (XVIII), en présence dudit catalyseur à base de métal.

16. Procédé conforme à l'une des revendications 10 à 12, dans lequel, dans l'étape (12), on fait d'abord réagir le composé de formule (XVIII) avec un métal, de préférence avec un métal alcalino-terreux tel que du magnésium, et l'on fait ensuite réagir le produit de Grignard ainsi obtenu avec le composé de formule (XII), en présence dudit catalyseur à base de métal.

17. Procédé conforme à l'une des revendications 1 à 12, 15 et 16, dans lequel, dans l'étape (i2), on fait réagir le composé de formule (X) avec un composé choisi dans l'ensemble formé par le chlorure de tertiobutyl-diméthyl-silyle, le chlorure de triméthyl-silyle, le chlorure de triéthyl-silyle, le chlorure de triisopropyl-silyle, et le chlorure de tertiobutyl-diphényl-silyle.

18. Procédé conforme à l'une des revendications 1 à 12 et 15 à 17, dans lequel on réalise l'étape (j2) en présence d'un composé choisi dans l'ensemble constitué par la 2-hydroxy-pyridine, les N-hydroxy-carboxamides, les N-hydroxy-carboximides tels que le N-hydroxy-succinimide, et les mélanges de tels composés.

19. Procédé conforme à la revendication 18, dans lequel, dans l'étape (j2), on utilise comme solvant une amine tertiaire comme de la triméthyl-amine et/ou de la triéthyl-amine.

20. Procédé de préparation de l'aliskirène, de formule (I) suivante, ou d'un sel pharmacologiquement admissible de ce composé : 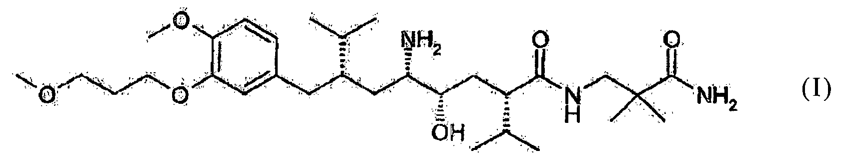 lequel procédé comporte :
le fait de convertir un composé de formule (II) : 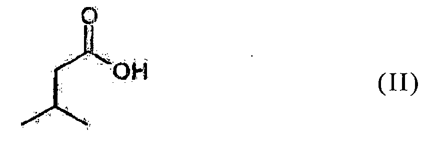 en un composé de formule (III) : 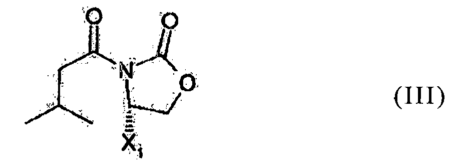 dans laquelle X₁ représente un groupe alkyl-aryle,
et le fait de convertir ce composé de formule (III) en un composé de formule (I).

21. Procédé de préparation de l'aliskirène, de formule (I) suivante, ou d'un sel pharmacologiquement admissible de ce composé : 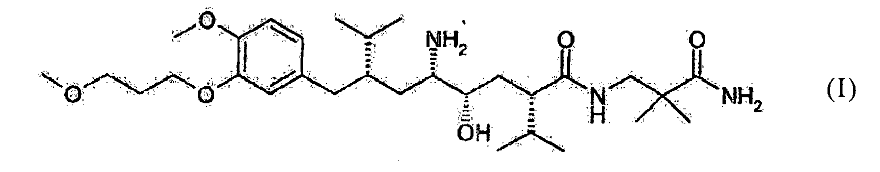 lequel procédé comporte :
le fait de faire réagir un composé de formule (III) : 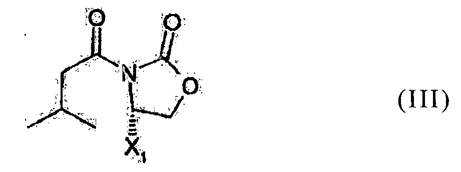
dans laquelle X₁ représente un groupe alkyl-aryle,
avec un composé de formule 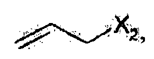 dans laquelle X₂ représente un atome d'halogène,
de manière à obtenir un composé de formule (IV) : 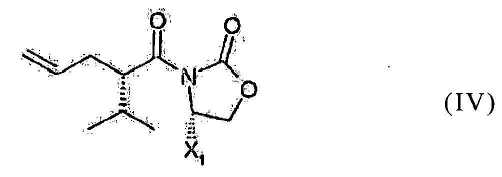 et le fait de convertir ce composé de formule (IV) en un composé de formule (I).

22. Procédé de préparation de l'aliskirène, de formule (I) suivante, ou d'un sel pharmacologiquement admissible de ce composé : 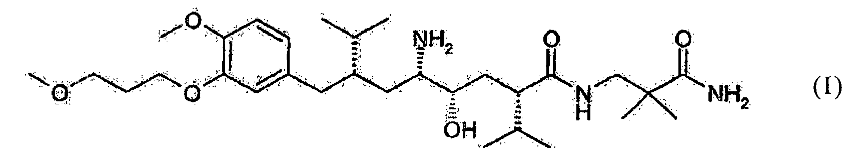 lequel procédé comporte :
le fait de convertir un composé de formule (IV) : 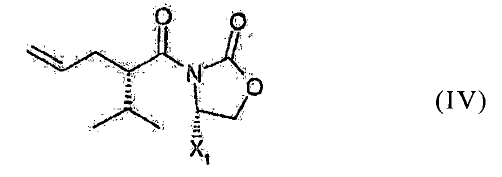
dans laquelle X₁ représente un groupe alkyl-aryle,
en un composé de formule (V) : 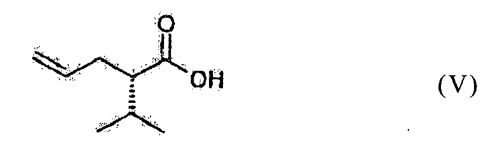 et le fait de convertir ce composé de formule (V) en un composé de formule (I).

23. Procédé de préparation de l'aliskirène, de formule (I) suivante, ou d'un sel pharmacologiquement admissible de ce composé : 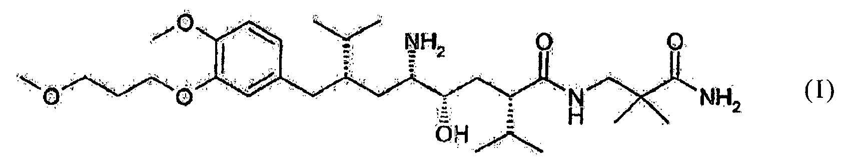 lequel procédé comporte :
le fait de convertir un composé de formule (V) : 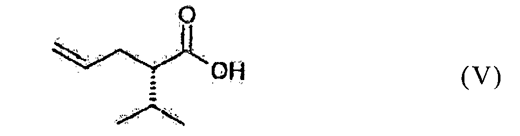
en un composé de formule (VI) : 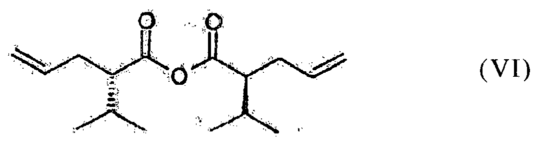 et le fait de convertir ce composé de formule (VI) en un composé de formule (I).

24. Procédé de préparation de l'aliskirène, de formule (I) suivante, ou d'un sel pharmacologiquement admissible de ce composé : 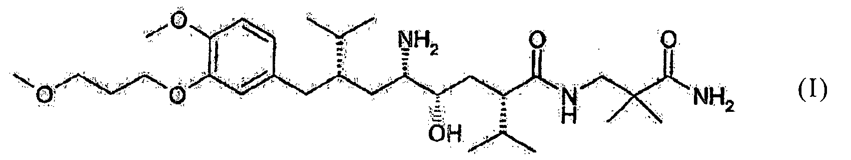 lequel procédé comporte :
le fait d'opérer la cyclisation d'un composé de formule (VI) : 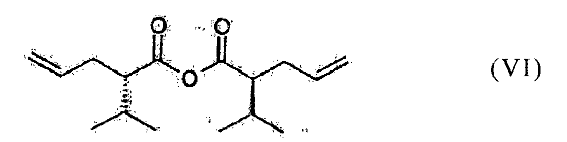 en présence d'un catalyseur à base de métal, de manière à obtenir un composé de formule (VII) : 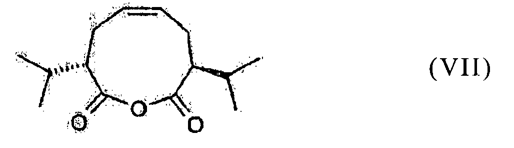 et le fait de convertir ce composé de formule (VII) en un composé de formule (I).

25. Procédé de préparation de l'aliskirène, de formule (I) suivante, ou d'un sel pharmacologiquement admissible de ce composé : 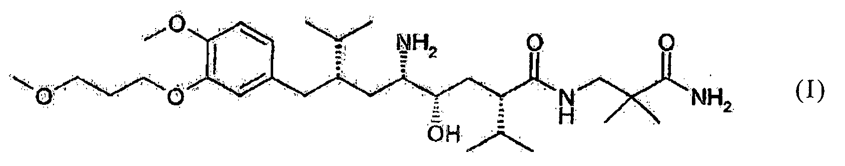 lequel procédé comporte :
le fait de convertir un composé de formule (VII) : 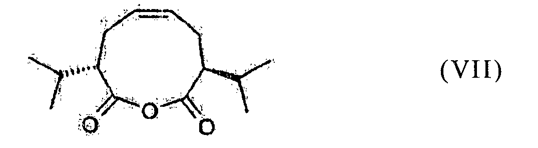 en un composé de formule (VIII) ou (VIIIA) : 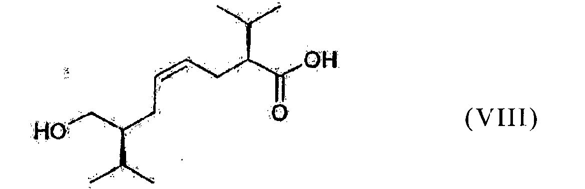 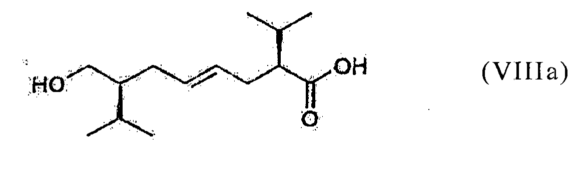 et le fait de convertir ce composé de formule (VIII) ou (VIIIa) en un composé de formule (I).

26. Procédé de préparation de l'aliskirène, de formule (I) suivante, ou d'un sel pharmacologiquement admissible de ce composé : 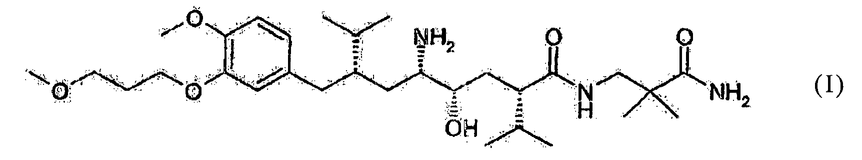 lequel procédé comporte :
le fait de convertir en lactone un composé de formule (VIII) ou (VIIIa) : 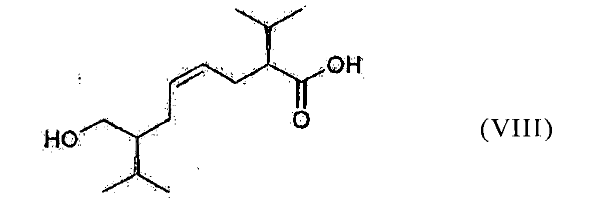 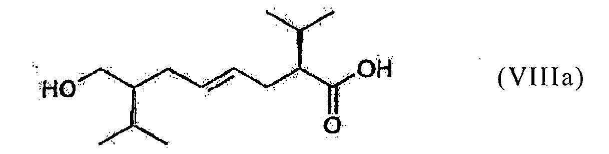
de manière à obtenir un composé de formule (IX) : 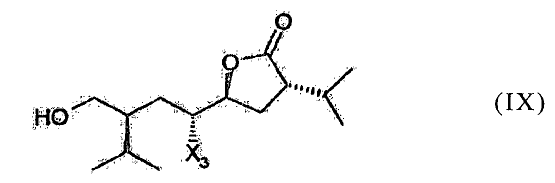 dans laquelle X₃ représente un atome d'halogène ;
et le fait de convertir ce composé de formule (IX) en un composé de formule (I).

27. Procédé de préparation de l'aliskirène, de formule (I) suivante, ou d'un sel pharmacologiquement admissible de ce composé : 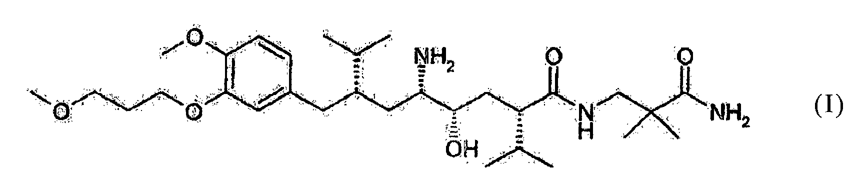 lequel procédé comporte :
le fait de convertir en azoture un composé de formule (IX) : 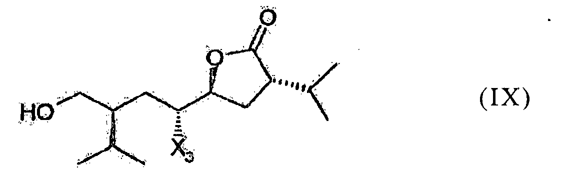
dans laquelle X₃ représente un atome d'halogène ; de manière à obtenir un composé de formule (X) : 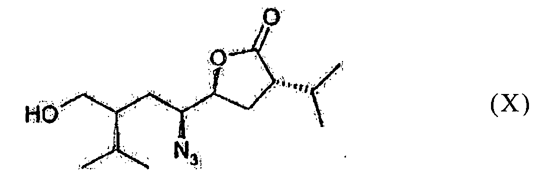 et le fait de convertir ce composé de formule (X) en un composé de formule (I).

28. Procédé de préparation de l'aliskirène, de formule (I) suivante, ou d'un sel pharmacologiquement admissible de ce composé : 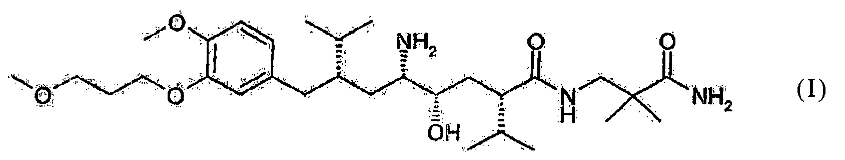 lequel procédé comporte :
le fait de convertir un composé de formule (X) : 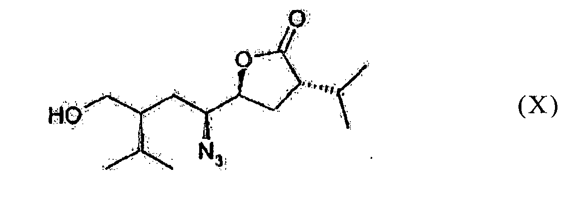
en un composé de formule (XI) : 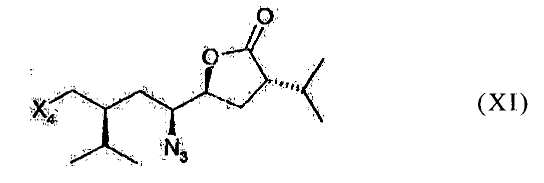 dans laquelle X₄ représente un atome d'halogène ;
et le fait de convertir ce composé de formule (XI) en un composé de formule (I).

29. Procédé de préparation de l'aliskirène, de formule (I) suivante, ou d'un sel pharmacologiquement admissible de ce composé : 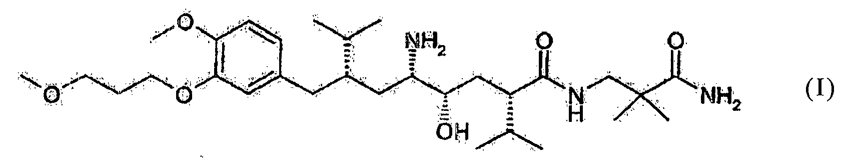 lequel procédé comporte :
le fait de faire réagir un composé de formule (XI) : 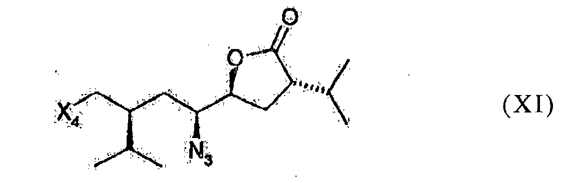
dans laquelle X₄ représente un atome d'halogène,
en présence d'un catalyseur à base de métal, avec un composé de formule (XII) : 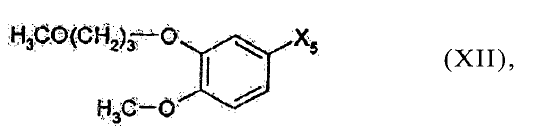 dans laquelle X₅ représente un atome d'halogène, de manière à obtenir un composé de formule (XIII) : 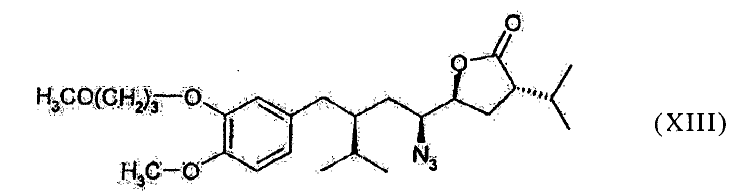 et le fait de convertir ce composé de formule (XIII) en un composé de
formule (I).

30. Procédé de préparation de l'aliskirène, de formule (I) suivante, ou d'un sel pharmacologiquement admissible de ce composé : 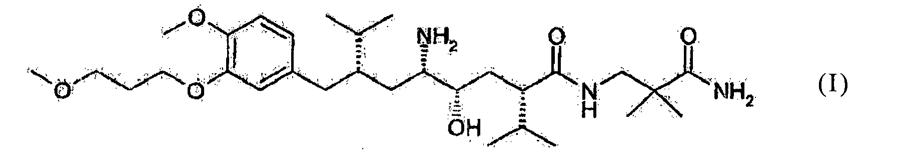 lequel procédé comporte :
le fait de convertir un composé de formule (X) : 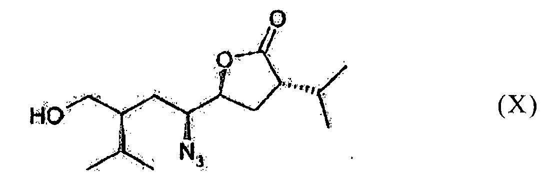
en un composé de formule (XVI) : 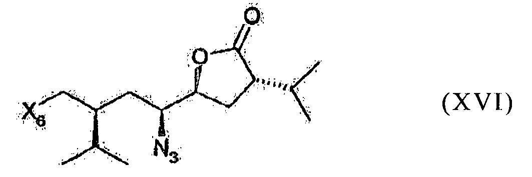 dans laquelle X₆ représente une entité choisie dans l'ensemble constitué par les groupes trialkyl-silyle, dialkyl-aryl-silyle, alkyl-diaryl-silyle et triaryl-silyle,
et le fait de convertir ce composé de formule (XVI) en un composé de formule (I).

31. Procédé de préparation de l'aliskirène, de formule (I) suivante, ou d'un sel pharmacologiquement admissible de ce composé : 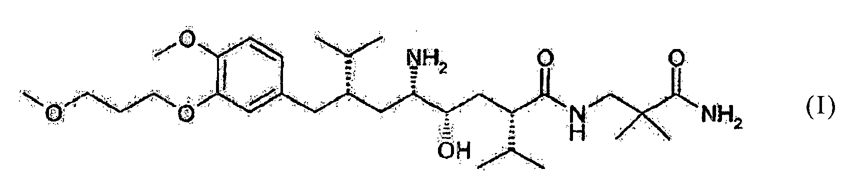 lequel procédé comporte :
le fait de faire réagir un composé de formule (XVI) : 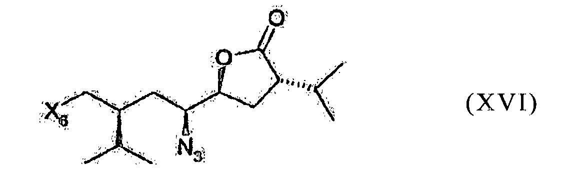
dans laquelle X₆ représente une entité choisie dans l'ensemble constitué par les groupes trialkyl-silyle, dialkyl-aryl-silyle, alkyl-diaryl-silyle et triaryl-silyle,
avec une amine de formule (XIV) : 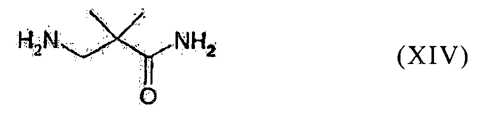 de manière à obtenir un composé de formule (XVII) : 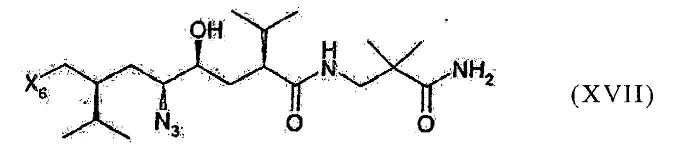 et le fait de convertir ce composé de formule (XVII) en un composé de formule (I).

32. Procédé de préparation de l'aliskirène, de formule (I) suivante, ou d'un sel pharmacologiquement admissible de ce composé : 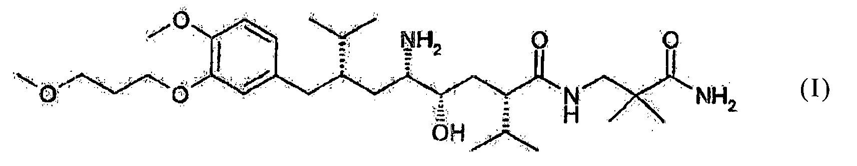 lequel procédé comporte :
le fait de convertir un composé de formule (XVII) : 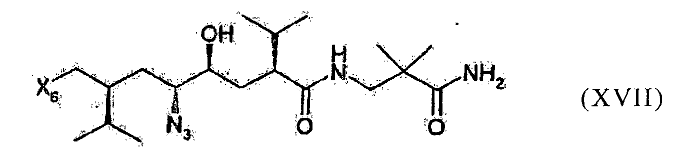
dans laquelle X₆ représente une entité choisie dans l'ensemble constitué par les groupes trialkyl-silyle, dialkyl-aryl-silyle, alkyl-diaryl-silyle et triaryl-silyle,
en un composé de formule (XVIII) : 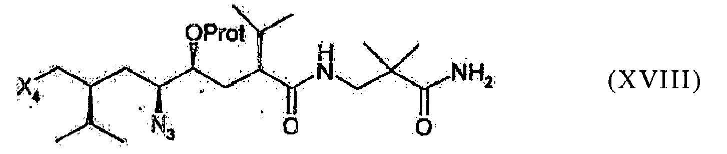 dans laquelle X₄ représente un atome d'halogène et Prot représente un groupe hydroxy-protecteur,
et le fait de convertir ce composé de formule (XVIII) en un composé de formule (I).

33. Procédé de préparation de l'aliskirène, de formule (I) suivante, ou d'un sel pharmacologiquement admissible de ce composé : 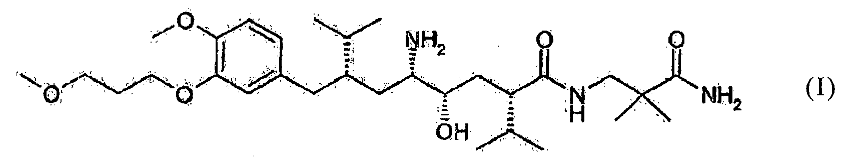 lequel procédé comporte :
le fait de faire réagir un composé de formule (XVIII) : 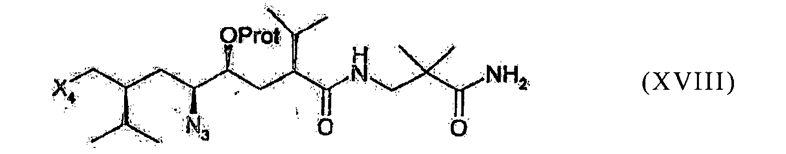
dans laquelle X₄ représente un atome d'halogène et Prot représente un groupe hydroxy-protecteur,
en présence d'un catalyseur à base de métal, avec un composé de formule (XII) : 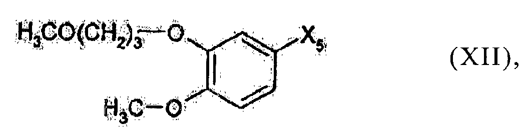 dans laquelle X₅ représente un atome d'halogène, de manière à obtenir un composé de formule (XIX) : 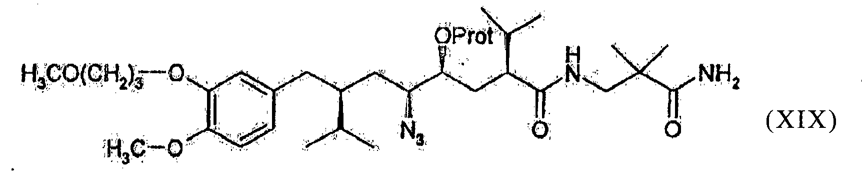 et le fait de convertir ce composé de formule (XIX) en un composé de formule (I).

34. Composé de formule (VI) :

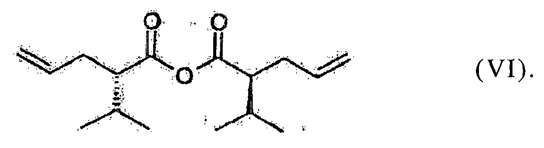

35. Composé de formule (VII) :

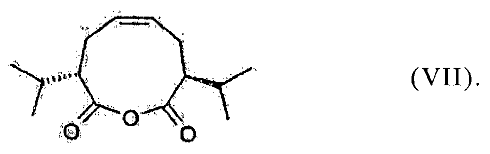

36. Composé de formule (VIII) ou (VIIIa) :

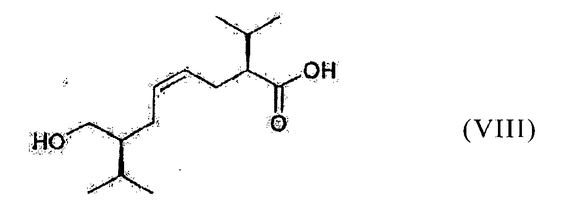

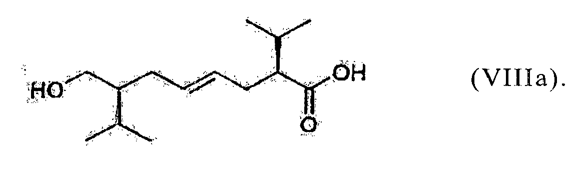

37. Composé de formule (IX) : 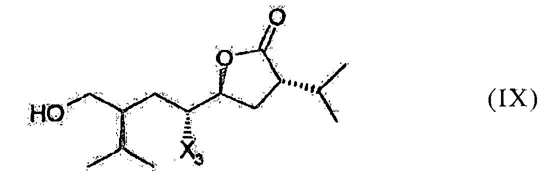 dans laquelle X₃ représente un atome d'halogène.

38. Composé de formule (XI) : 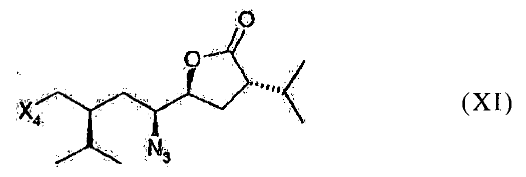 dans laquelle X₄ représente un atome d'halogène.

39. Composé de formule (XVI) : 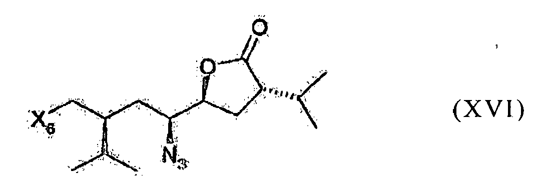 dans laquelle X₆ représente une entité choisie dans l'ensemble constitué par les groupes trialkyl-silyle, dialkyl-aryl-silyle, alkyl-diaryl-silyle et triaryl-silyle.

40. Composé de formule (XVII) : 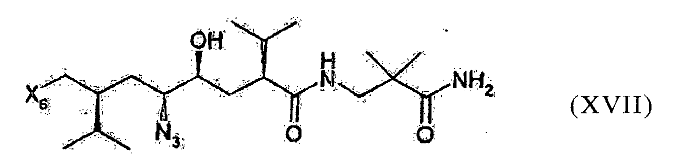 dans laquelle X₆ représente une entité choisie dans l'ensemble constitué par les groupes trialkyl-silyle, dialkyl-aryl-silyle, alkyl-diaryl-silyle et triaryl-silyle.

41. Composé de formule (XVIII) : 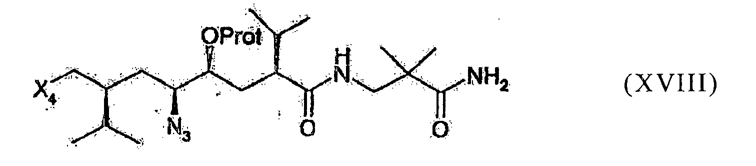 dans laquelle X₄ représente un atome d'halogène et Prot représente un groupe hydroxy-protecteur.

42. Utilisation d'un composé conforme à l'une des revendications 34 à 41 pour la préparation de l'aliskirène, de formule (I) suivante, ou d'un sel pharmacologiquement admissible de ce composé :

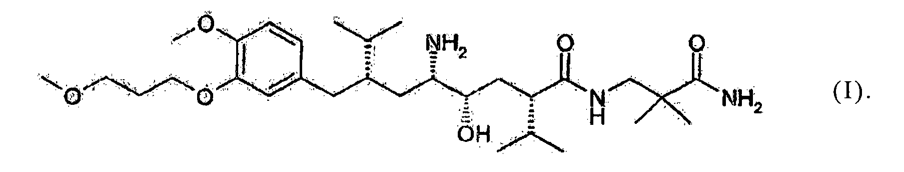

43. Procédé de préparation de l'aliskirène, de formule (I) suivante, ou d'un sel pharmacologiquement admissible de ce composé : 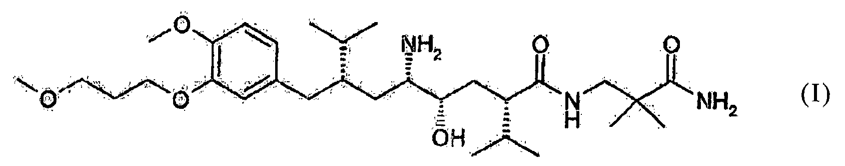 utilisant au moins un composé conforme à l'une des revendications 34 à 41.

44. Procédé de préparation d'une composition pharmaceutique contenant de l'aliskirène ou un sel pharmacologiquement admissible de ce composé, comportant le fait de préparer de l'aliskirène ou un sel pharmacologiquement admissible de ce composé, en opérant selon un procédé conforme à l'une des revendications 1 à 33 et 43, et le fait de transformer l'aliskirène ou son sel en une composition pharmaceutique.

45. Procédé conforme à la revendication 44, dans lequel on réalise la transformation en composition pharmaceutique de l'aliskirène, ou d'un sel pharmacologiquement admissible de ce composé, en opérant selon un procédé de granulation à l'état fondu à chaud et sans solvant.
